(19) Europäisches Patentamt
European Patent Office
Office européen des brevets

(11) EP 3 747 928 A1

(12) # EUROPEAN PATENT APPLICATION
published in accordance with Art. 153(4) EPC

(43) Date of publication:
**09.12.2020 Bulletin 2020/50**

(21) Application number: **19747294.7**

(22) Date of filing: **01.02.2019**

(51) Int Cl.:
***C08G 65/28*** *(2006.01)* ***A61K 31/7088*** *(2006.01)*
***A61K 47/34*** *(2017.01)* ***C08G 65/328*** *(2006.01)*

(86) International application number:
**PCT/JP2019/003617**

(87) International publication number:
**WO 2019/151478 (08.08.2019 Gazette 2019/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.02.2018 JP 2018016798**

(71) Applicant: **The University of Tokyo**
**Bunkyo-ku,**
**Tokyo 113-8654 (JP)**

(72) Inventors:
- **CABRAL, Horacio**
  **Tokyo 113-8654 (JP)**
- **UCHIDA, Satoshi**
  **Tokyo 113-8654 (JP)**
- **MIYAZAKI, Takuya**
  **Tokyo 113-8654 (JP)**

(74) Representative: **J A Kemp LLP**
**14 South Square**
**Gray's Inn**
**London WC1R 5JJ (GB)**

(54) **POLYMER COMPOUND FOR NUCLEIC ACID DELIVERY**

(57) A polymer compound selected from a homopolymer of (a) and a block copolymer of (b). (a) A homopolymer comprising a polyether segment having a side chain including a primary amine. (b) A block copolymer of a polyether segment having a side chain including a primary amine, and a poly(ethylene glycol) chain.

Fig. 1

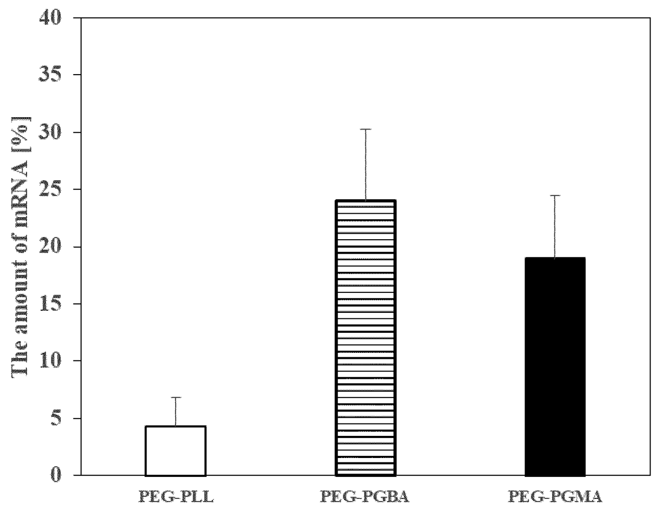


EP 3 747 928 A1

## Description

FIELD OF THE INVENTION

The present invention is associate with the technical field of delivery of nucleic acid to targeted cells or tissues. Particularly, the present invention is related to the development of novel cationic polymers as a carrier for nucleic acid delivery.

BACKGROUND ART

Since mRNA is translated into therapeutic proteins in the cytoplasm, it has a potential for nucleic acid therapeutics. While plasmid DNA, one of nucleic acid therapeutics, can induce insertion into the host genomic DNA and require delivery systems targeting to the cell nucleus, mRNA holds advantages over such plasmid DNA (for example, Non-patent document 1). However, systemic administration of naked mRNA shows rapid enzymatic degradation due to negatively charged phosphate group, poor cellular uptake and unfavorable immune responses (For example, Non-patent document 2). Thus, the development of nanocarriers loading mRNA is essential for application of mRNA.

Polyion complex (PIC) micelles are one of the promising nanocarriers capable of delivering mRNA. Block copolymers comprising poly(ethylene glycol) and polycation can encapsulate mRNA *via* electrostatic interactions to protect mRNA payload in the PIC core (for example, Non-patent document 3). mRNA-loaded PIC micelles comprising poly(amino acids) is utilized to inhibit enzymatic degradation of loaded mRNA, and to enhance the cellular uptake *via* charge neutralization, resulting in the achievement of augmented gene expression (for example, Non-patent document 3).

However, further improvement of PIC stability in harsh environment *in vivo* is important for *in vivo* applications.

PRIOR ART DOCUMENTS

Non-patent documents

Non-patent document 1: U. Sahin et. al., Nat. Rev. Drug Discovery 13 (2014), 759-780

Non-patent document 2: N. B. Tsui et. al., Clin. Chem. 48 (2002), 1647-1653

Non-patent document 3: S. Uchida et. al., Biomaterials 82 (2016), 221-228


SUMMARY OF THE INVENTION

Problem to be Solved by the Invention

This invention is aimed to develop a polymeric system for delivering nucleic acids such as RNA to targeted cells.

To develop a new nucleic acid delivery system, the inventors have designed a block copolymer comprising poly(ethylene glycol) and polyether amine, and introduced a primary amine into a side chain of the polyether segment, thereby accomplishing the present invention.

Means for Solving Problem

Thus, the present invention is as follows.

(1) A polymer compound selected from a homopolymer of (a) and a block copolymer of (b) below:

(a) a homopolymer comprising a polyether segment having a side chain containing a primary amine;
(b) a block copolymer comprising a polyether segment having a side chain containing a primary amine and a poly(ethylene glycol) chain.

(2) The polymer compound according to (1), wherein the homopolymer is represented by Formula I below:

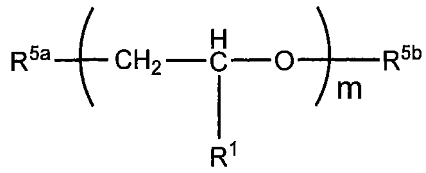

(I)

(wherein, $R^1$ represents a primary amine linked *via* a methylene group or an ester bond, $R^{5a}$ and $R^{5b}$ each independently represent a hydrogen atom, a halogen atom or a group represented by $-OCOCH(NH_2)R^3$ (wherein, $R^3$ represents a hydrogen atom or a group represented by:

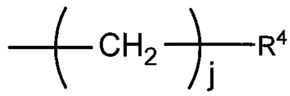

(wherein, $R^4$ represents an optionally substituted hydrocarbon group having a carbon number of 1-20 or an optionally substituted heterocyclic functional group, and j represents an integer of 0-5),
and m represents an integer of 2-5,000).
(3) The polymer compound according to (2), wherein the primary amine linked *via* a methylene group or an ester bond is represented by the following formula:

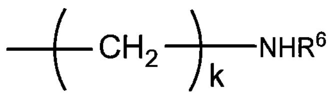
or
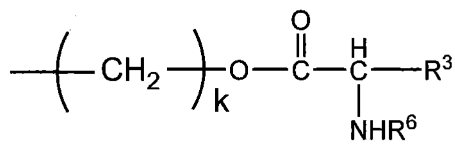

(wherein, $R^3$ represents a hydrogen atom or a group represented by:

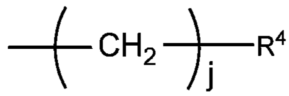

(wherein, $R^4$ represents an optionally substituted hydrocarbon group having a carbon number of 1-20 or an optionally substituted heterocyclic functional group, and j represents an integer of 0-5),
or $R^3$ and $R^4$ each independently represent a side chain structure of an arbitrary amino acid of a protein, $R^6$ represents a hydrogen atom or one or more arbitrary amino acids, and k represents an integer of 0-5).

(4) The polymer compound according to (1), wherein the block copolymer is represented by Formula II below:

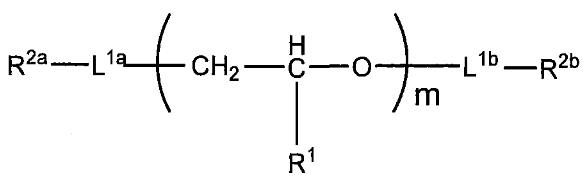

(II)

[wherein, $R^1$ represents a primary amine linked *via* a methylene group or an ester bond, $R^{2a}$ and $R^{2b}$ each independently represent a hydrogen atom or a group represented by:

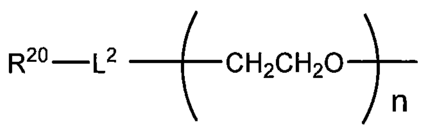

(wherein, $R^{20}$ represents a group represented by $-O-CH_3$ or a ligand molecule, $L^2$ represents a linking group,

and n represents an integer of 5-20,000),
$L^{1a}$ and $L^{1b}$ each independently represent a linking group, and m represents an integer of 2-5,000].

(5) The polymer compound according to (4), wherein the primary amine linked *via* a methylene group or an ester bond is represented by the following formula:

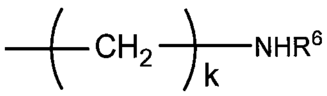
or
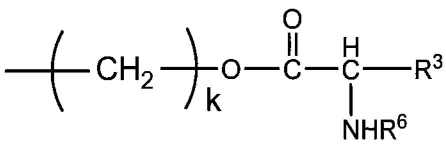

(wherein, $R^3$ represents a hydrogen atom or a group represented by:

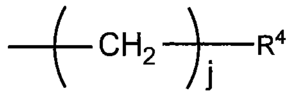

(wherein, $R^4$ represents an optionally substituted hydrocarbon group having a carbon number of 1-20 or an optionally substituted heterocyclic functional group, and j represents an integer of 0-5),
or $R^3$ and $R^4$ each independently represent a side chain of an arbitrary amino acid, $R^6$ represent a hydrogen atom or one or more arbitrary amino acids, and k represents an integer of 0-5).

(6) The compound according to any one of (2)-(5), wherein $R^1$ is represented by $-CH_2NH_2$ or $-CH_2CH_2CH_2CH_2NH_2$.
(7) The compound according to any one of (2)-(5), wherein $R^1$ is represented by:

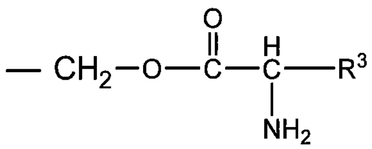

(wherein, $R^3$ represents a hydrogen atom or a group represented by:

$-CH_2-R^4$

(wherein, $R^4$ represents a propyl group, an optionally substituted indolyl group or an optionally substituted phenyl group).
(8) The compound according to any one of (4)-(7), wherein $L^{1a}$ and/or $L^{1b}$ is represented by $-CH_2CH_2O-$.
(9) The compound according to any one of (4)-(8), wherein $R^2$ is represented by:

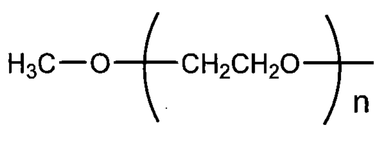

(wherein, n represents an integer of 5-20,000).
(10) A polyion complex comprising the polymer compound according to any one of (1)-(7) and nucleic acids.
(11) The polyion complex according to (10), wherein the nucleic acids are RNA or DNA.
(12) The polyion complex according to (11), wherein RNA is siRNA, mRNA, antisense RNA, RNA aptamer, self-replicating RNA, miRNA (micro RNA) or IncRNA (long non-coding RNA).
(13) The polyion complex according to (11), wherein DNA is antisense DNA, DNA aptamer, pDNA (plasmid DNA) or MCDNA (minicircle DNA).
(14) A kit for preparing a device to deliver nucleic acids to targeted cells or tissues, the kit comprising the polymer compound according to any one of (1)-(9).
(15) A device for delivering nucleic acids to targeted cells or tissues, the device comprising the polyion complex according to any one of (10)-(13).

(16) A method for producing a block copolymer comprising a polyether segment having a side chain containing a primary amine and a poly(ethylene glycol) chain, the method comprising: obtaining a block copolymer comprising poly(ethylene glycol) and a polyether segment having a side chain by dehydrating polyethylene glycol and then carrying out epoxy ring-opening polymerization; and introducing a primary amine into the side chain of the polyether segment.
(17) The method according to (16), wherein dehydration is performed using toluene.
(18) The method according to either one of (16) and (17), wherein epoxy ring-opening polymerization is carried out using 1,2-epoxy-5-hexene or epichlorohydrin.

EFFECT OF THE INVENTION

The present invention has enabled clinical applications of RNA therapeutics. For example, siRNA can suppress expression of a disease-related gene *in vivo,* and mRNA can sustainably and safely produce therapeutic proteins. Moreover, the present invention can significantly supress enzymatic degradation of RNA and augment transfection efficiency of RNA.

BRIEF DESCRIPTION OF THE DRAWINGS

Figure 1 shows results of the stability of polymeric micelles comprising PEG-PLL, PEG-PGBA, PEG-PGMA and PEG-PGLeu against enzymatic degradation.
Figure 2 shows results of transfection efficacy into cultured cells using PEG-PLL, PEG-PGBA and PEG-PGMA.
Figure 3 shows results of the stability against heparin as polyanion using PEG-PLL, PEG-PGBA, PEG-PGMA.
Figure 4 shows results of the stability against heparin as polyanion using PEG-PGGly, PEG-PGLeu, PEG-PGTry and PEG-PGTyr.
Figure 5 shows results of *in vivo* bioavailability using PEG-PLL, PEG-PGBA and PEG-PGMA.
Figure 6 shows results of hydrolysis capability using PEG-PLL and PEG-PGLeu.
Figure 7 shows results of *in vitro* cytotoxicity against cultured cells using PEG-PLL and PEG-PGLeu.
Figure 8 shows heat of reaction related to PIC formation.
Figure 9 shows fluorescence intensities of tryptophan residues in PEG-PGTrp.
Figure 10 shows fluorescence intensities of tyrosine residues in PEG-PGTyr.
Figure 11 shows the stability of mRNA-loaded micelles against enzymatic degradation in serum.
Figure 12 shows transfection efficacy of gene encoding luciferase into cultured cells using mRNA-loaded micelles.
Figure 13 shows blood circulation of mRNA-loaded micelles.
Figure 14 shows transfection efficacy of luciferase gene using mRNA-loaded micelles.
Figure 15 shows transfection efficacy of luciferase gene using mRNA-loaded micelles.
Figure 16 shows the amount of primary amines in polymers under physiological salt conditions.
Figure 17 shows *in vitro* cytotoxicity of polymers against cultured cells.

MODE FOR CARRYING OUT THE INVENTION

The present invention is related to a block copolymer comprising poly(ethylene glycol) and poly(glycidyl amine), or a homopolymer comprising poly(glycidyl amine), wherein a primary amine is introduced into the side chain of the polyether segment.

The present inventors focused on the chemical structure of the main chain of the block copolymer. While a polyion complex comprising double-stranded nucleic acids with low flexibility (e.g., plasmid DNA) and poly(amino acids) with low flexibility showed no change in the entropy before and after the formation of the polymeric micelles, entropy greatly decreased with a polyion complex comprising single-stranded nucleic acids with high flexibility (e.g., mRNA) and poly(amino acids) with low flexibility, presumably because the movement of mRNA is greatly restricted by the poly(amino acids) after the formation of the polymeric micelles (for example, K. Hayashi et. al., Macromol. Rapid Commun. 37 (2016), 486-493).

Hence, the present inventors assumed that a highly flexible block copolymer may improve the mobility of mRNA after the formation of the polymeric micelles and suppress the decrease of entropy. Thus, a highly flexible block copolymer was designed by engineering the chemical structure of the main chain to be polyether segment with ether bond. Moreover, a block copolymer having a small number of methylene groups at the side chain of the polycation chain was designed to confirm the influence of the number of methylene groups at the side chain on stability. Furthermore, a biodegradable polymer was designed with a view to *in vivo* medical applications. Specifically, an amino acid was introduced into the

side chain structure of the polyether segment *via* a biodegradable ester bond.

1. Polymer compound

The present invention is a polymer compound selected from a homopolymer of (a) and a block copolymer of (b) below.

(a) a homopolymer comprising a polyether segment having a side chain containing a primary amine; and
(b) a block copolymer comprising a polyether segment having a side chain containing a primary amine and a poly(ethylene glycol) chain.

The polymer compound of the present invention is, for example, one represented by Formula I or II below.

(a) A homopolymer comprising a polyether segment having a side chain containing a primary amine:
a homopolymer represented by Formula I below:

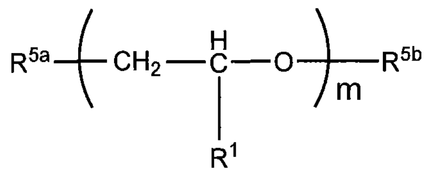

(I)

(wherein, $R^1$ represents a primary amine linked *via* a methylene group or an ester bond, $R^{5a}$ and $R^{5b}$ each independently represent a hydrogen atom, a halogen atom or -OCOCH(NH2)R3 (wherein, $R^3$ represents a hydrogen atom, or a group represented by:

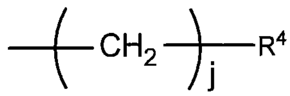

(wherein, $R^4$ represents an optionally substituted hydrocarbon group having a carbon number of 1-20 or an optionally substituted heterocyclic functional group, and j represents an integer of 0-5),
and m represents an integer of 2-5,000).

In the polymer represented by Formula I above, the primary amine linked *via* a methylene group or the primary amine linked *via* an ester bond may, for example, be one represented by the following formula:

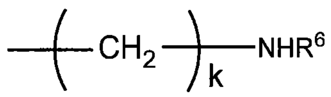

or

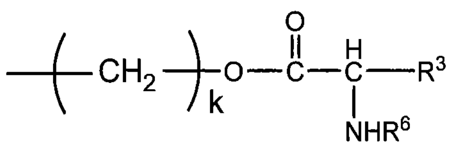

(wherein, $R^3$ represents a hydrogen atom or a group represented by:

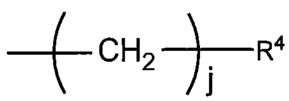

(wherein, $R^4$ represents an optionally substituted hydrocarbon group having a carbon number of 1-20 or an optionally substituted heterocyclic functional group, and j represents an integer of 0-5)
or $R^3$ and $R^4$ each independently represent a side chain structure of an arbitrary amino acid of a protein, $R^6$ represents a hydrogen atom or one or more arbitrary amino acids, and k represents an integer of 0-5).

$R^3$ and $R^4$ are, for example, a side chain structure of an arbitrary amino acid of a protein (for example, the

twenty kinds of amino acids).

Furthermore, according to the present invention, $R^6$ in $-NHR^6$ may be an arbitrary amino acid (for example, the twenty kinds of natural amino acids or synthetic amino acids) to form a dipeptide, a tripeptide, a tetrapeptide or the like. Moreover, if $R^6$ is one or more arbitrary amino acids, they may be added through formation of an amide bond.

For example, cysteine, histidine or other basic amino acid (for example, lysine, arginine) may alternatively be used in place of leucine or tryptophan of PGLeu or PGGTry described in the examples. Cysteine allows disulfide crosslinking whereas histidine can introduce the capability of endosomal escape to the polymer. Basic amino acids can increase cationic charges. Alternatively, a dipeptide having tryptophan coupled with PGLeu (PGLeu-Trp), a dipeptide having cysteine coupled with PGLeu (PGLeu-Cys), or a dipeptide having histidine coupled with PGLeu (PGLeu-His) can also be employed.

Once an arbitrary amino acid is conjugated, the NH moiety of $-NHR^6$ is no longer a primary amine, but since the coupled amino acid has a primary amine, the "primary amine linked *via* an ester bond" according to the present invention also comprises these compounds.

According to the present invention, $R^6$ in $-NHR^6$ comprises one represented by $-(COCR^3NH)_p-H$. $R^3$ is the same as described above. p is a repeating unit representing an integer of 1-10,000.

Herein, optionally substituted heterocyclic functional groups include an indolyl ring, a pyrrolidone group, a furan ring, a pyridine ring, a morpholine ring, an epoxy ring, a purine ring and a pyrimidine ring.

In addition, examples of the halogen atom in Formula I include F, Cl, Br and I.

In the polymer represented by Formula I, $R^{5a}$ and $R^{5b}$ can be Br (bromine) when $R^1$ is a primary amine linked *via* methylene groups, or can be $-OCOCH(NH)R_3$ group when $R^1$ is a primary amine linked *via* ester bonds.

(b) A block copolymer comprising a polyether segment having a side chain containing a primary amine and a poly(ethylene glycol) chain:

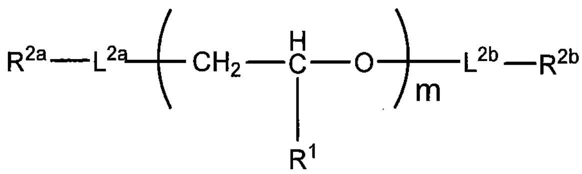

(II)

In Formula II, $R^1$ represents a primary amine linked *via* a methylene group or a primary amine linked *via* an ester bond where the detailed chemical structure of these primary amines are the same as described above.

$R^{2a}$ and $R^{2b}$ each independently represent a hydrogen atom or a hydrophilic group. Examples of the hydrophilic group include a group represented by:

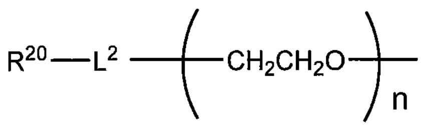

(where $R^{20}$ represents functional groups like $-O-CH_3$ or a ligand molecule, $L^2$ represents a linking group, and n represents an integer of 5-20,000) (for example, poly(ethylene glycol)).

In addition to the groups represented by the formula above (poly(ethylene glycol)), $R^{2a}$ and $R^{2b}$ can be other hydrophilic groups (hydrophilic polymers). Examples of such hydrophilic groups include poly(ethylenimine), 2-methacryloyloxyethyl phosphorylcholine, 2-acryloyloxyethyl phosphorylcholine, N-(2-methacrylamide)ethyl phosphorylcholine and 4-methacryloyloxybutyl phosphorylcholine.

A ligand molecule refers to a compound that can be used for targeting a specific biomolecule, where examples of ligands include an antibody, a protein, an amino acid, a small molecule and a monomer of a biomolecules.

According to the present invention, $L^{1a}$ and $L^{1b}$ each independently represent linkage groups, and m represents an integer of 2-5,000. A linking group refers to a spacer between the respective blocks of a block copolymer, which may be, for example, a single bond or a group represented by $-CH_2CH_2O-$.

Examples of $R^1$ include those represented by $-CH_2NH_2$ and $-CH_2CH_2CH_2CH_2NH_2$. In addition, examples of $R^1$ include those represented by:

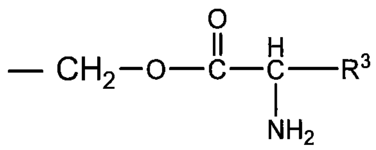

(wherein $R^3$ represents a hydrogen atom, or a group represented by:

$-CH_2-R^4$

(wherein $R^4$ represents a propyl group, an optionally substituted indolyl group or an optionally substituted phenyl group)).

Examples of $R^{2a}$ and/or $R^{2b}$ include those represented by the following formula:

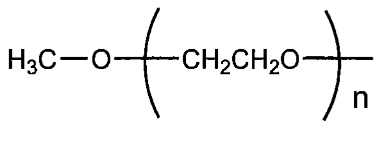

(wherein n represents an integer of 5-20,000).

<About hydrocarbon group with substituents>

The hydrocarbon group is a saturated or unsaturated, noncyclic or cyclic hydrocarbon group. In the case of the noncyclic hydrocarbon group, it can be either linear or branched. Examples of the hydrocarbon group include an alkyl group with a carbon number of 1-20 (hereinafter referred to as "$C_{1-20}$", which similarly applies to other carbon numbers as well), a $C_{2-20}$ alkenyl group, a $C_{2-20}$ alkynyl group, a $C_{4-20}$ alkyldienyl group, a $C_{6-18}$ aryl group, a $C_{7-20}$ alkylaryl group, a $C_{7-20}$ arylalkyl group, a $C_{3-20}$ cycloalkyl group, a $C_{3-20}$ cycloalkenyl group, a $C_{1-20}$ alkoxy group, a $C_{6-20}$ aryloxy group, a $C_{7-20}$ alkylaryloxy group and a $C_{2-20}$ alkoxycarbonyl group.

Examples of the $C_{1-6}$ alkyl group include a methyl group, an ethyl group, a propyl group, an isopropyl group, a n-butyl group, a sec-butyl group, a tert-butyl group, a pentyl group and a hexyl group.

Examples of the $C_{1-20}$ alkyl group include a methyl group, an ethyl group, a propyl group, an isopropyl group, a n-butyl group, a sec-butyl group, a tert-butyl group, a pentyl group, a hexyl group, an octyl group, a nonyl group, a decyl group, an undecyl group and a dodecyl group.

Examples of the $C_{2-20}$ alkenyl group include a vinyl group, an allyl group, a propenyl group, an isopropenyl group, a 2-methyl-1-a propenyl group, a 2-methylallyl group and a 2-butenyl group.

Examples of the $C_{2-20}$ alkynyl group include an ethynyl group, a propynyl group and a butynyl group.

Examples of the $C_{4-20}$ alkyldienyl group include a 1,3-butadienyl group.

Examples of the $C_{6-18}$ aryl group include a phenyl group, a 1-naphthyl group, a 2-naphthyl group, an indenyl group, a biphenyl group, an anthryl group and a phenanthryl group.

Examples of the $C_{7-20}$ alkylaryl group include an o-tolyl group, a m-tolyl group, a p-tolyl group, a 2,3-xylyl group, a 2,5-xylyl group, an o-cumenyl group, a m-cumenyl group, a p-cumenyl group and a mesityl group.

Examples of the $C_{7-20}$ arylalkyl group include a benzyl group, a phenethyl group, a 1-naphthylmethyl group, a 2-naphthylmethyl group, a 1-phenylethyl group, a phenylpropyl group, a phenylbutyl group, a phenylpentyl group, a phenylhexyl group, a methylbenzyl group, a dimethylbenzyl group, a trimethylbenzyl group, an ethylbenzyl group, a methylphenethyl group, a dimethylphenethyl group and a diethylbenzyl group.

Examples of the $C_{3-20}$ cycloalkyl group include a cyclopropyl group, a cyclobutyl group, a cyclopentyl group, a cyclohexyl group, a cycloheptyl group and a cyclooctyl group.

Examples of the $C_{3-20}$ cycloalykenyl group include a cyclopropenyl group, a cyclobutenyl group, a cyclopentenyl group, a cyclopentadienyl group and a cyclohexenyl group.

Examples of the $C_{1-20}$ alkoxy group include a methoxy group, an ethoxy group, a propoxy group, a butoxy group and a pentyloxy group.

Examples of the $C_{6-20}$ aryloxy group include a phenyloxy group, a naphtyloxy group and a biphenyloxy group.

Examples of the $C_{7-20}$ alkylaryloxy group include a methyl phenyloxy group, an ethyl phenyloxy group, a propyl phenyloxy group, a butyl phenyloxy group, a dimethyl phenyloxy group, a diethyl phenyloxy group, a dipropyl phenyloxy group, a dibutyl phenyloxy group, a methylethyl phenyloxy group, a methylpropyl phenyloxy group and an ethylpropyl phenyloxy group.

Examples of the $C_{2-20}$ alkoxycarbonyl group include a methoxycarbonyl group, an ethoxycarbonyl group, a 2-methoxycarbonyl group and a t-butoxycarbonyl group.

Specific examples of the substituent of the optionally substituted hydrocarbon group and the optionally substituted heterocyclic functional group include a halogen atom (F, Cl, Br, I), a hydroxy group, a thiol group, a nitro group, a cyano group, a formyl group, a carboxyl group, an amino group, a silyl group, a methanesulfonyl group, a $C_{1-6}$ alkyl group, a $C_{2-6}$ alkenyl group, a $C_{2-6}$ alkynyl group, a $C_{3-8}$ cycloalkyl group, a $C_{6-10}$ aryl group, a $C_{1-6}$ alkoxy group, a $C_{1-7}$ acyl group and a $C_{2-7}$ alkoxycarbonyl group.

According to the present invention, an optionally substituted phenyl group or an optionally substituted heterocyclic functional group is preferable but the present invention is not limited to polymer compounds with such a substituent.

While the molecular weight (Mn) of the polymer represented by Formula I is not limited, it can be 200-500,000 and preferably 1,000-100,000.

Moreover, while the molecular weight (Mn) of the block copolymer represented by Formula II is not limited, it can be 400-1,400,000 and preferably 1,000-144,000. Furthermore, for each segment in block copolymers, the molecular weight of the PEG segment can be 200-200,000, and preferably 200-44,000. The molecular weight of the polyether segment can be 200-500,000, and more preferably 1,000-100,000.

## 2. Production of polymer compound

### (1) Production of polymer represented by Formula I

A method for producing a polymer represented by Formula I will be described as below:
A homopolymer comprising a polyether segment having a side chain was obtained by epoxy ring-opening polymerization using tetra-n-octylammonium bromide as an initiator. The kind of ring-opening polymerization is not limited as long as the monomer is an epoxide. For example, epichlorohydrin, 1,2-epoxy-5-hexene, 1-allyl-2,3-epoxypropane, epibromohydrin, 3,4-epoxy-1-butane, 1,2-epoxy-9-decene, 2,3-epoxypropyl propargyl ether or the like can be used.

After the polymerization, the molecular weight distribution and the degree of polymerization of the obtained block copolymer are preferably analyzed.

Furthermore, in order to prepare PIC micelles through an electrostatic interaction with RNA, a primary amine is introduced into the side chain of the polyether segment of the obtained block copolymer. Introduction of a primary amine into the side chain of the polyether segment can be achieved with high yield by using the favorable solvent for hydroboration-amination. The resulting polyether having the primary amine in the side chain can be referred to as poly(glycidyl amine).

### (2) Production of polymer represented by Formula II

A method to synthesize a polymer represented by Formula II will be described as below.

As for the chemical synthesis of a block copolymer, the poly(ethylene glycol) as an initiator is considered to interfere with the polymerization due to its hygroscopic property. Accordingly, poly(ethylene glycol) is azeotropically dehydrated prior to polymerization. Subsequently, epoxy ring-opening polymerization is carried out to obtain a block copolymer comprising poly(ethylene glycol) and a polyether segment having a side chain. In this case, poly(ethylene glycol) is dehydrated with toluene or the like to proceed the epoxy ring-opening polymerization for producing a block copolymer with controlled molecular weight distribution and the degree of polymerization. The epoxy ring-opening polymerization is not particularly limited as long as the polymerization monomer has a substructure of epoxy groups. For example, epichlorohydrin, 1,2-epoxy-5-hexene, 1-allyl-2,3-epoxypropane, epibromohydrin, 3,4-epoxy-1-butane, 1,2-epoxy-9-decene, 2,3-epoxypropyl propargyl ether or the like can be used.

After the polymerization, the molecular weight distribution and the degree of polymerization of the obtained block copolymer are preferably analyzed.

Furthermore, in order to prepare polymeric micelles through an electrostatic interaction with RNA, a primary amine is introduced into the side chain of the polyether segment of the obtained block copolymer. Introduction of a primary amine into the side chain of the polyether segment can be achieved with high yield by selecting the favorable solvent for hydroboration-amination. The resulting polyether having the primary amine in the side chain can be referred to as poly(glycidyl amine).

Specific examples of the polymer compound represented by Formula II of the present invention include the polymers described in Production examples 2-7 as below.

## 3. Polyion complex

Polyion complex micelles (polymer complex) of the present invention are supramolecular assemblies that can be obtained by mixing nucleic acids and polymers of the present invention in buffer, which is also called a polyion complex (PIC) or a polyion complex-type polymeric micelle (PIC micelle).

If the polymer utilized to form polyion complex micelles of the present invention is a block copolymer comprising PEG and poly(glycidyl amine), the nucleic acids and the polycation can assembly *via* electrostatic interaction to form a core-shell structure with the PEG shell and nucleic acid payload in the core.

The polymer complex of the present invention can be prepared by mixing, for example, nucleic acids and the polymer compound in an arbitrary buffer.

The PIC of the present invention have a size of, for example, approximately 60-80 nm in a diameter as measured by dynamic light scattering (DLS).

The mixing ratio of the polymer compound and the nucleic acids is not limited. For example, the ratio of the total number (N) of cationic groups (for example, amino groups) in the block copolymer to the total number (P) of phosphate groups in the nucleic acids (N/P ratio) is preferably 1-30, more preferably 1-10 and still more preferably 1-3.

For a specific example of this production method, see the synthesis scheme described in the examples as below.

The functionality of the polyion complex of the present invention as a transport carrier of the nucleic acids can be assessed by quantifying stability in the serum and the efficiency of translation to produce protein. Moreover, stability against polyanions that are abundant in living body or their function as transporting carriers in *in vivo* environments can be assessed using blood retention as an indicator.

In another aspect of the present invention, a biodegradable block copolymer that has an amino acid introduced into the side chain of the polyether segment *via* an ester bond can be designed as a biodegradable nanocarrier with a view to clinical applications. In this case, *in vivo* cytotoxicity of the polymer can be reduced by cleavage of the ester bonds. Cleavage of the ester bonds can be assessed by quantifying the primary amine in the polymer under physiological saltconditions. The polymer toxicity can also be assessed by using cultured cells.

For example, the functionality of a biodegradable polymer with one of the amino acids, leucine, as a transport carrier for RNA delivery can be investigated by the stability against polyanions abundantly existing *in vivo* and the stability in serum. In this case, the isobutyl group in leucine enhances the stability by hydrophobic interaction in the polyion complex.

Furthermore, when the stability of the micelles of the present invention in the serum was assessed, the micelles showed significant stability and thus higher translation efficiency than micelles comprising polylysine chains. Moreover, since the stability against polyanions abundantly existing *in vivo* was also enhanced, the micelle is expected to show high stability *in vivo*. The bioavailability in *in vivo* environments was assessed by detecting intact mRNA, which is rapidly degraded by enzymatic attack. As a result, the micelle of the present invention showed prolonged circulation in blood stream than the micelle comprising polylysine chains. This shows that introduction of polyether segment having an ether bond with low strain can enhance the resistance of mRNA against enzymatic degradation *in vivo*.

In regard to a biodegradable nanocarriers, when the cleavage of the ester bond between the polymer and the amino acid was assessed under physiological salt conditions, the decrease of the primary amine in association with hydrolysis was confirmed, and cytotoxicity against cultured cells was reduced. In addition, when the stability of polymeric micelles comprising biodegradable polymers with one of the amino acids, in this case, leucine against polyanion exchange was assessed, they enhanced the stability more than the formulation without hydrophobic functional groups, in this case, isobutyl groups. Hence, the present inventors succeeded in developing nanocarriers with high stability and low cytotoxicity.

## 4. Nucleic acids

In one aspect, nucleic acids encapsulated in polyion complex micelles of the present invention as a component of the core moiety are DNA or RNA. Examples of RNA include mRNA, siRNA (small interfering RNA), antisense nucleic acids (antisense RNA), an aptamer (RNA aptamer), self-replicating RNA, miRNA (micro RNA) and IncRNA (long non-coding RNA). Examples of DNA include antisense nucleic acids (antisense DNA), an aptamer (DNA aptamer), pDNA (plasmid DNA) and MCDNA (minicircle DNA).

Any siRNA can be used as long as it can suppress the expression of the targeted gene through RNA interference (RNAi), where examples of the target gene favorably include, but not limited to, a cancer (tumor) gene, an anti-apoptosis gene, a cell cycle-related gene and a growth signal gene. Moreover, the base pair of siRNA is generally not limited as long as it is less than 30 base pairs (for example, 19-21 base pairs).

Since nucleotide acids such as siRNA are anionic molecules, it can interact (assemble) with the polycation block of the block copolymer *via* electrostatic interaction.

## 5. Kit for a nucleic acid delivery device

A kit for a nucleic acid delivery device of the present invention is characterized by comprising a polymer compound of the present invention. This kit can preferably be used, for example, for gene therapy using RNAi for a target cell and protein therapy using mRNA.

In the kit of the present invention, the preserved form of the polymer is not limited, and it can be selected from a solution form, a powdery form or the like considering its stability (storability), ease of use, and else.
In addition to the above-described polymer compound, the kit of the present invention may include other components. Examples of other components include nucleic acids to transfect into the cells, buffers used for dissolving, diluting and the like, protein and an instruction (instruction manual), which may suitably be selected according to the purpose of use and the type of polymer.
The kit of the present invention is used for preparing polyion complex (PIC) micelles which encapsulate nucleic acids (for example, mRNA or siRNA) in the core to transfect into targeted cells. The prepared PIC holds advantages as a device for delivering nucleic acids to targeted cells.

6. Device for delivering nucleic acids

The present invention can provide a nucleic acid delivery device comprising the above-described polyion complex. The delivery device of the present invention is capable of stabilizing nucleic acids which had been difficult to stably deliver to targeted cells by enhancing the resistance against enzyme degradation.
The delivery device of the present invention can be applied to various animals including, but not limited to, humans, mice, rats, rabbits, pigs, dogs, cats and the like. A parenteral method such as intravenous injection is usually employed for administering the device to a subject animal, where various conditions including the amount, number and period of administration are suitably determined according to the type and the state of the subject animal.
The delivery device of the present invention can be used for a treatment where desired nucleic acids are transfected into cells in pathological sites (gene therapy). Hence, the present invention can also provide a pharmaceutical composition comprising the above-described polyion complex for treating various diseases, gene therapeutics for various diseases which comprises pharmaceutical composition, and a method for treating various diseases which comprises use of PIC (particularly, gene therapy).
Methods and conditions are the same as described above. Moreover, examples of the various diseases include, but not limited to, cancers (for example, lung cancer, pancreas cancer, brain tumor, liver cancer, breast cancer, colorectal cancer, neuroblastoma and bladder cancer), cardiovascular diseases, musculoskeletal diseases and central nervous system diseases.
The above-described pharmaceutical composition can be formulated by an ordinary method by suitably selecting and using a diluent, a filler, a bulking agent, a binder, a wetting agent, a disintegrant, a lubricant, a surfactant, a dispersant, a buffer, a preservative, a solubilizing aid, an antiseptic, a flavoring agent, a soothing agent, a stabilizer, a tonicity adjusting agent and the like which are generally used for drug production. In addition, an intravenously injectable agent (including intravenous drip) is usually employed as the form of the pharmaceutical composition. For example, the pharmaceutical composition may be provided in a single-dose ampoule or in a multiple-dose container.

EXAMPLES

Hereinafter, the present invention will be described more specifically by showing examples. The scope of the present invention, however, should not be limited to these examples.

Example 1

Synthesis of respective polymers

Production example 1: Synthesis of poly(ethylene glycol)-block-poly(L-lysine)

Synthesis scheme:

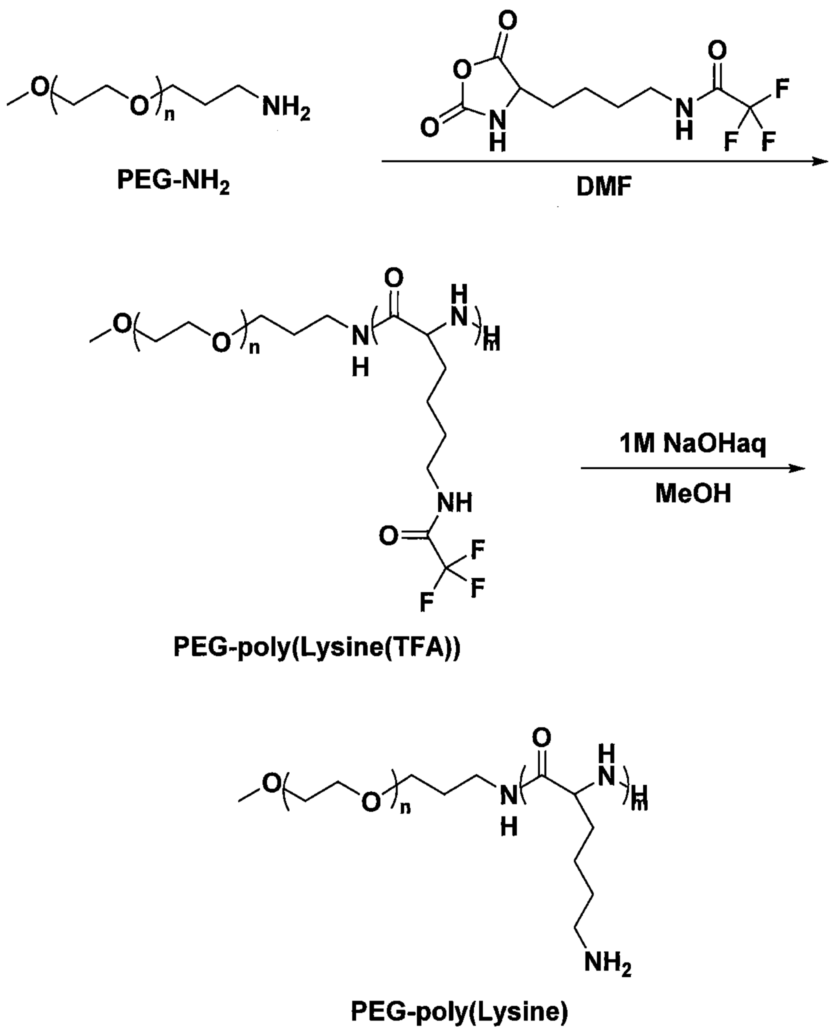


PEG-NH2

PEG-poly(Lysine(TFA))

PEG-poly(Lysine)

Poly(ethylene glycol) having an amino group at the end was dehydrated by vacuum drying while ε-trifluoroacetic acid-L-lysine-N-carboxylic anhydride (Lys(TFA)-NCA) as a monomer was dissolved in N,N-dimethylformamide (DMF), and they were subjected to the polymerization at 35°C overnight. After the reaction, purification was conducted by reprecipitation with diethyl ether, which was followed by vacuum drying to give a powdery polymer. Next, the polymer was dissolved in methanol, to which an aqueous sodium hydroxide solution was added to allow reaction at room temperature overnight. After the reaction, the resultant was purified by dialysis against water and then lyophilized to give a powdery polymer. The resulting polymer (hereinafter, also referred to as PEG-PLL) was a polymer that could be represented by the following structural formula, where n = 272 and m = 83.

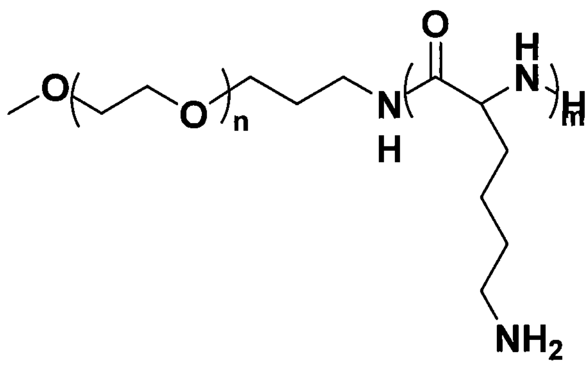

Production example 2: Synthesis of poly(ethylene glycol)-block-poly(glycidyl butyl amine)

Synthesis scheme:

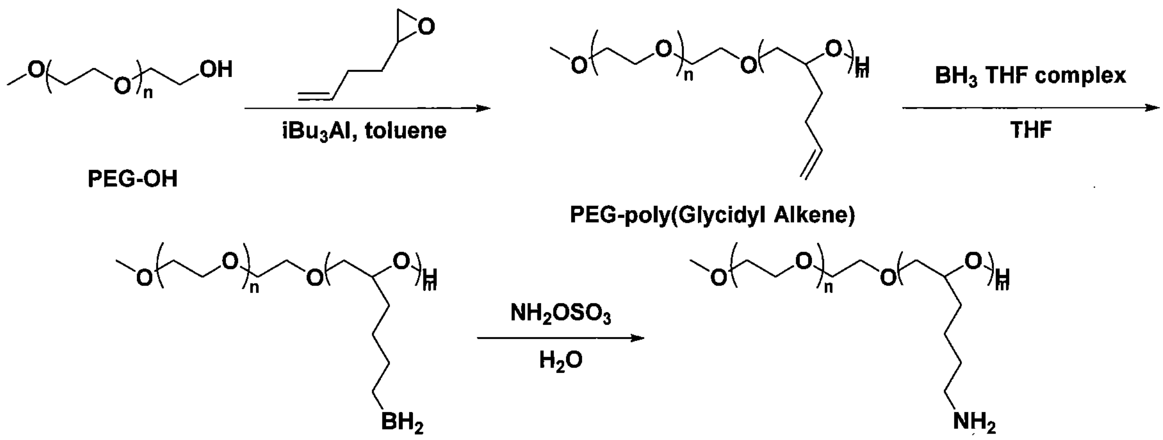

PEG-OH
PEG-poly(Glycidyl Alkene)
PEG-poly(Glycidyl Borane)
PEG-poly(Glycidyl Butyl Amine)
(PEG-PGBA)

Poly(ethylene glycol) having a hydroxyl group at the end was dissolved in toluene and the resultant was heated at 180°C for an hour to dehydrate the poly(ethylene glycol). To this, 1,2-epoxy-5-hexene as a polymerization monomer and triisobutyl aluminum as a catalyst were added to allow polymerization at room temperature overnight. After the reaction, purification was conducted by reprecipitation with diethyl ether, which was followed by vacuum drying to give a powdery polymer. Next, the polymer was dissolved in tetrahydrofuran, to which borane was added and the resultant was allowed to reflux at 66°C overnight. To this, powdery hydroxylamine-O-sulfonic acid was added to allow reaction at room temperature overnight. After the reaction, the resultant was purified by dialysis against water and then lyophilized to give a powdery polymer. The resulting polymer (hereinafter, also referred to as PEG-PGBA) was a polymer that could be represented by the following structural formula, where n = 272 and m = 93.

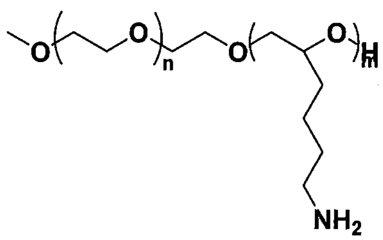

Production example 3: Synthesis of poly(ethylene glycol)-block-poly(glycidyl methyl amine)

Synthesis scheme:

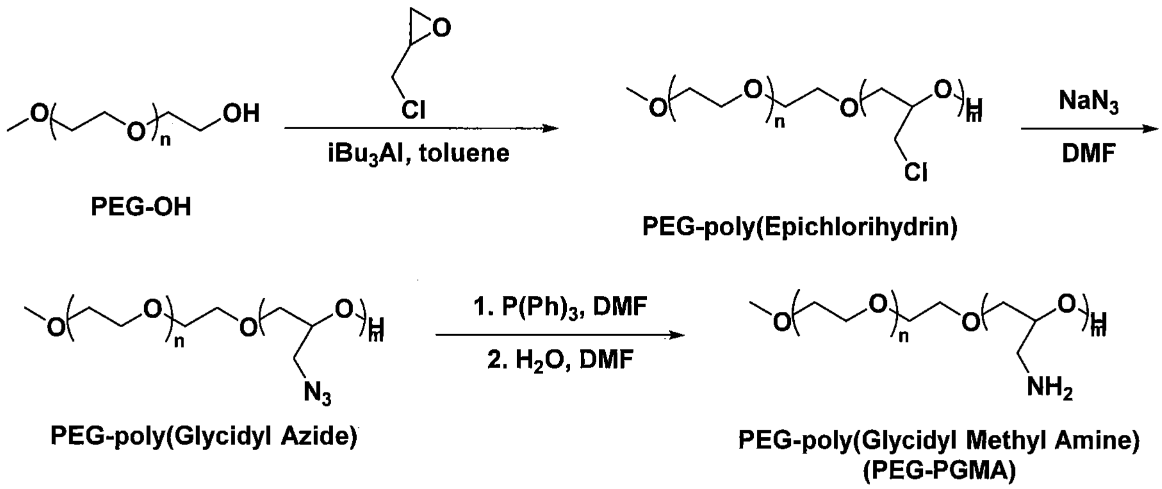

PEG-OH
PEG-poly(Epichlorihydrin)
PEG-poly(Glycidyl Azide)
PEG-poly(Glycidyl Methyl Amine)
(PEG-PGMA)

Poly(ethylene glycol) having a hydroxyl group at the end was dissolved in toluene, and the resultant was heated at 180°C for an hour to dehydrate the poly(ethylene glycol). To this, epichlorohydrin as a monomer and triisobutyl aluminum as a catalyst were added to allow polymerization at room temperature overnight. After the reaction, purification was conducted by reprecipitation with diethyl ether, which was followed by vacuum drying to give a powdery polymer. Next, the polymer was dissolved in DMF, to which sodium azide was added to allow reaction at 180°C overnight. After the reaction, purification was conducted by reprecipitation with diethyl ether, which was followed by vacuum drying to give a powdery polymer. Furthermore, the polymer was dissolved in DMF, to which triphenylphosphine was added to allow reaction at room temperature overnight. After the reaction, water was added to allow reaction overnight, after which the resultant was purified by dialysis against water and lyophilized to give a powdery polymer. The resulting polymer (hereinafter, also referred to as PEG-PGMA) was a polymer that could be represented by the following structural formula, where n = 272 and m = 81.

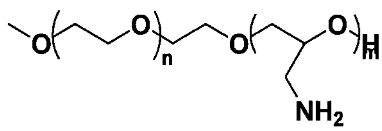

Production example 4: Synthesis of polyethylene glycol)-block-poly(glycidyl glycine)

Synthesis scheme:

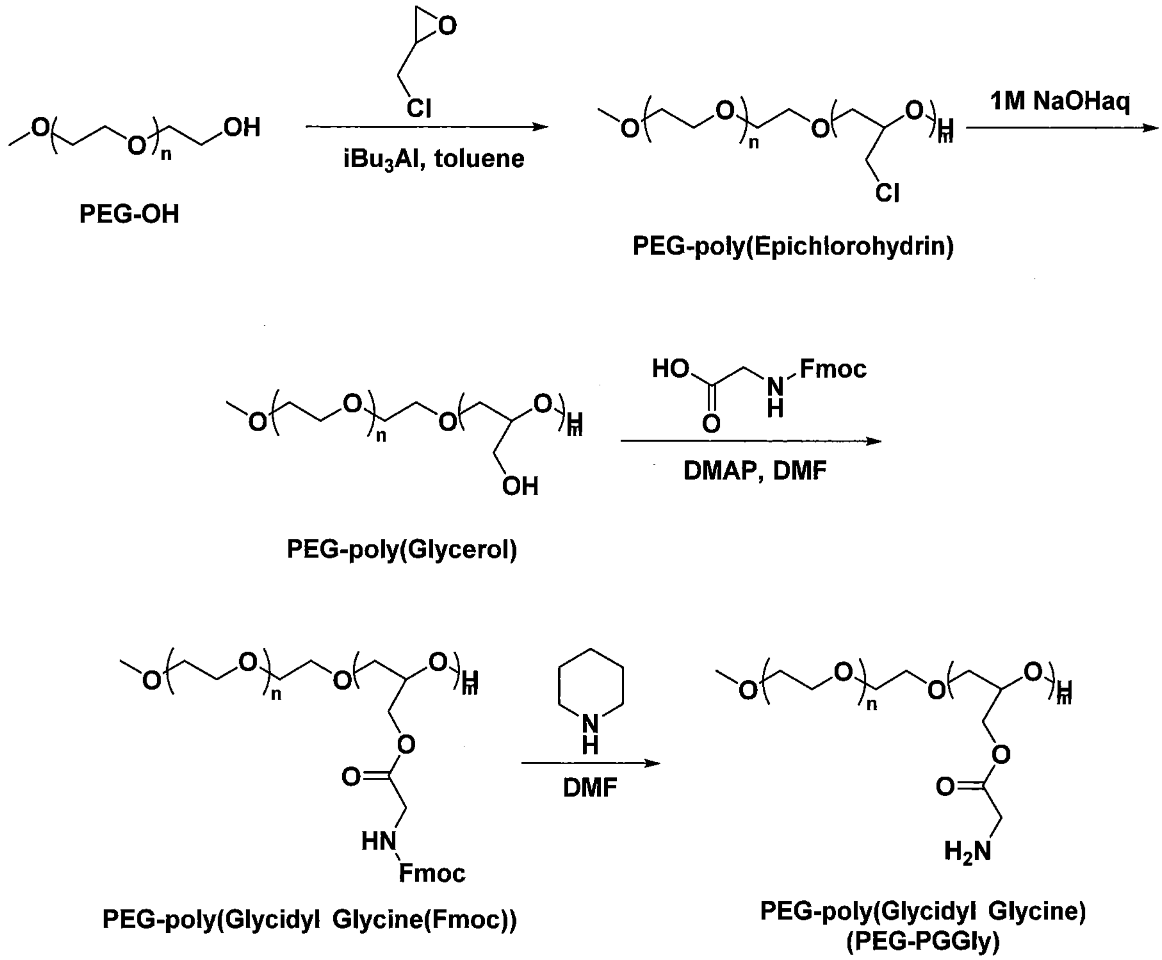


PEG-OH

PEG-poly(Epichlorohydrin)

PEG-poly(Glycerol)

PEG-poly(Glycidyl Glycine(Fmoc))

PEG-poly(Glycidyl Glycine) (PEG-PGGly)

Poly(ethylene glycol) having a hydroxyl group at the end was dissolved in toluene, and the resultant was heated at 180°C for an hour to dehydrate the poly(ethylene glycol). To this, epichlorohydrin as a monomer and triisobutyl aluminum as a catalyst were added to allow polymerization at room temperature overnight. After the reaction, purification was conducted by reprecipitation with diethyl ether, which was followed by vacuum drying to give a powdery polymer. Next, an aqueous sodium hydroxide solution was added to allow reaction overnight, after which the resultant was purified by dialysis against water and then lyophilized to give a powdery polymer. The polymer was further dissolved in DMF, to which N-[(9H-fluorene-9-ylmethoxy)carbonyl]-L-glycine, dimethylaminopyridine and 1-(3-dimethylaminopropyl)-3-ethylcarbodiimide were added to allow reaction overnight. After the reaction, purification was conducted by reprecipitation with diethyl ether, which was followed by vacuum drying to give a powdery polymer. Next, the polymer was dissolved in DMF, to which piperidine was added to allow reaction overnight. After the reaction, the resultant was purified by dialysis against water and then lyophilized to give a powdery polymer. The resulting polymer (hereinafter, also referred to as PEG-PGGly) was a polymer that could be represented by the following structural formula, where n = 272 and m = 81.

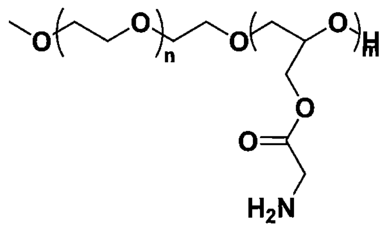

Production example 5: Synthesis of poly(ethylene glycol)-block-poly(glycidyl leucine)

Synthesis scheme:

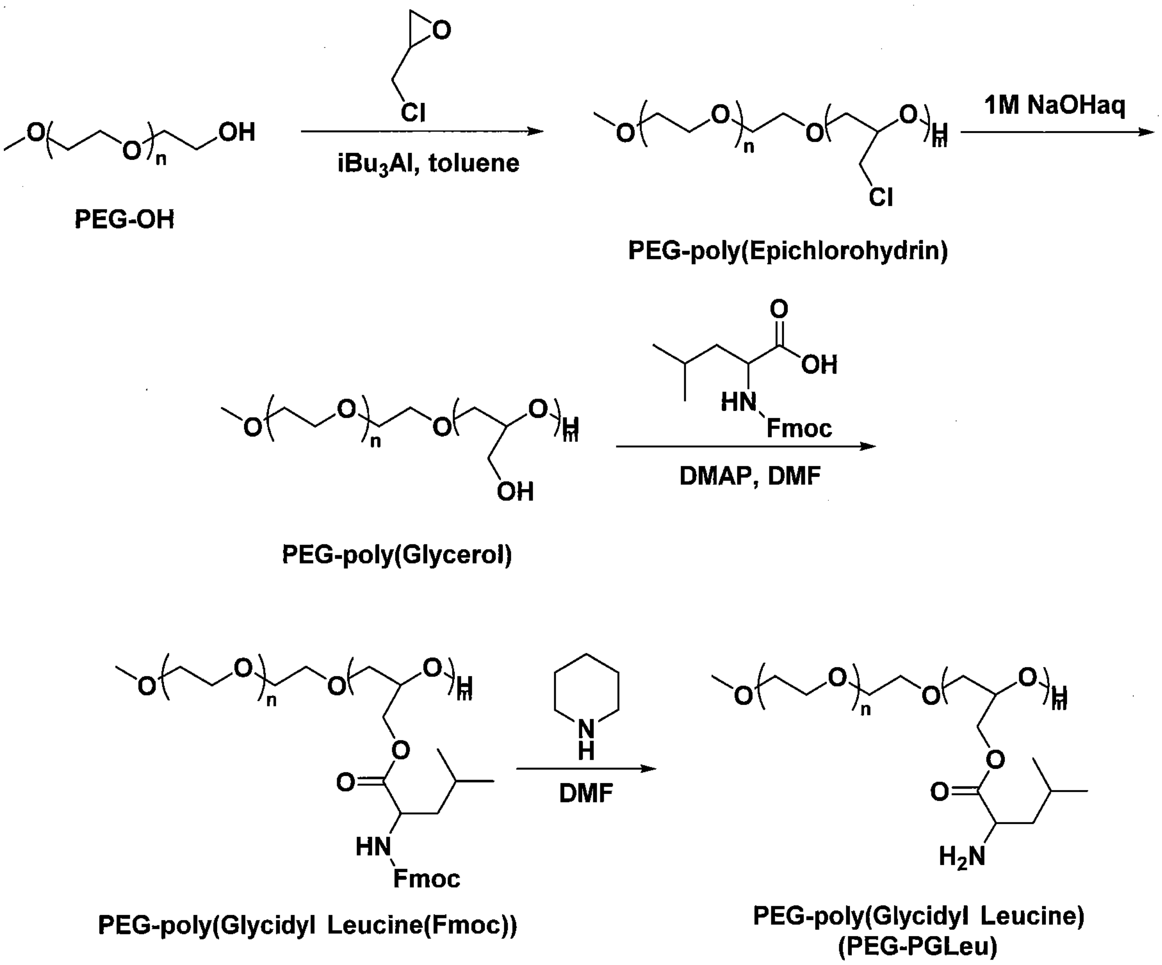


PEG-OH

PEG-poly(Epichlorohydrin)

PEG-poly(Glycerol)

PEG-poly(Glycidyl Leucine(Fmoc))

PEG-poly(Glycidyl Leucine) (PEG-PGLeu)

Poly(ethylene glycol) having a hydroxyl group at the end was dissolved in toluene, and the resultant was heated at 180°C for an hour to dehydrate the poly(ethylene glycol). To this, epichlorohydrin as a monomer and triisobutyl aluminum as a catalyst were added to allow polymerization at room temperature overnight. After the reaction, purification was conducted by reprecipitation with diethyl ether, which was followed by vacuum drying to give a powdery polymer. Next, an aqueous sodium hydroxide solution was added to allow reaction overnight, after which the resultant was purified by dialysis against water and then lyophilized to give a powdery polymer. The polymer was further dissolved in DMF, to which N-[(9H-fluorene-9-ylmethoxy)carbonyl]-L-leucine, dimethylaminopyridine and 1-(3-dimethylaminopropyl)-3-ethylcarbodiimide were added to allow reaction overnight. After the reaction, purification was conducted by reprecipitation with diethyl ether, which was followed by vacuum drying to give a powdery polymer. Next, the polymer was dissolved in DMF, to which piperidine was added to allow reaction overnight. After the reaction, the resultant was purified by dialysis against water and then lyophilized to give a powdery polymer. The resulting polymer (hereinafter, also referred to as PEG-PGLeu) was a polymer that could be represented by the following structural formula, where n = 272 and m = 81.

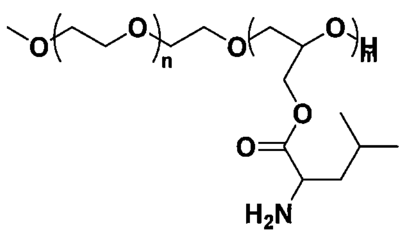

Production example 6: Synthesis of poly(ethylene glycol)-block-poly(glycidyl tryptophan)

Synthesis scheme:

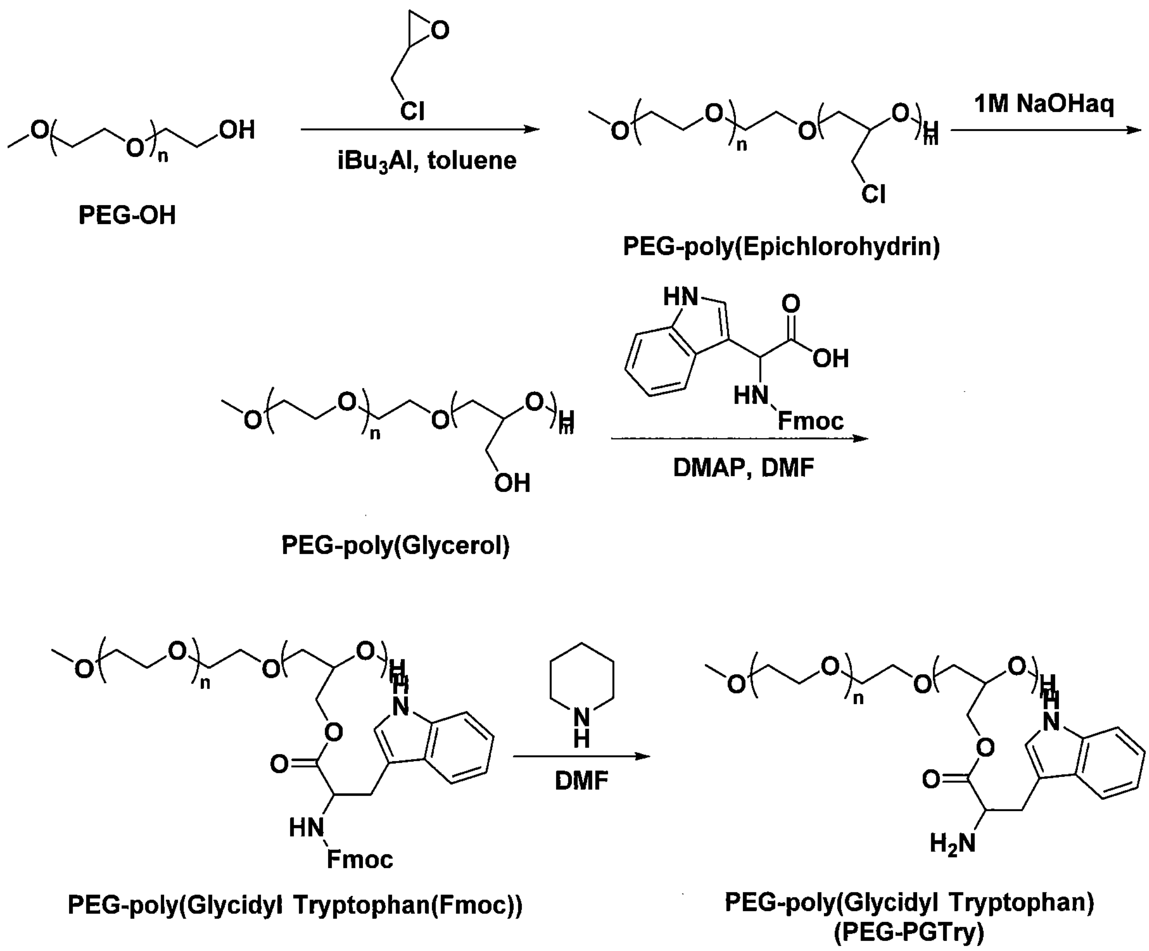


Poly(ethylene glycol) having a hydroxyl group at the end was dissolved in toluene, and the resultant was heated at 180°C for an hour to dehydrate the poly(ethylene glycol). To this, epichlorohydrin as a monomer and triisobutyl aluminum as a catalyst were added to allow polymerization at room temperature overnight. After the reaction, purification was conducted by reprecipitation with diethyl ether, which was followed by vacuum drying to give a powdery polymer. Next, an aqueous sodium hydroxide solution was added to allow reaction overnight, after which the resultant was purified by dialysis against water and lyophilized to give a powdery polymer. The polymer was further dissolved in DMF, to which N-[(9H-fluorene-9-ylmethoxy)carbonyl]-L-tryptophan, dimethylaminopyridine and 1-(3-dimethylaminopropyl)-3-ethylcarbodiimide were added to allow reaction overnight. After the reaction, purification was conducted by reprecipitation with diethyl ether, which was followed by vacuum drying to give a powdery polymer. Next, the polymer was dissolved in DMF, to which piperidine was added to allow reaction overnight. After the reaction, the resultant was purified by dialysis against water and then lyophilized to give a powdery polymer. The resulting polymer (hereinafter, also referred to as PEG-PGTry) was a polymer that could be represented by the following structural formula, where n = 272 and m = 81.

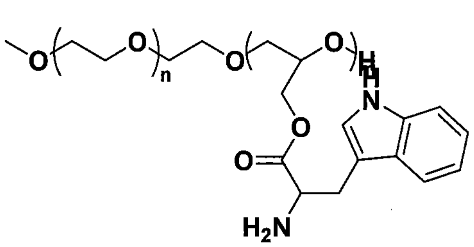

Production example 7: Synthesis of poly(ethylene glycol)-block-poly(glycidyl tyrosine)

Synthesis scheme:

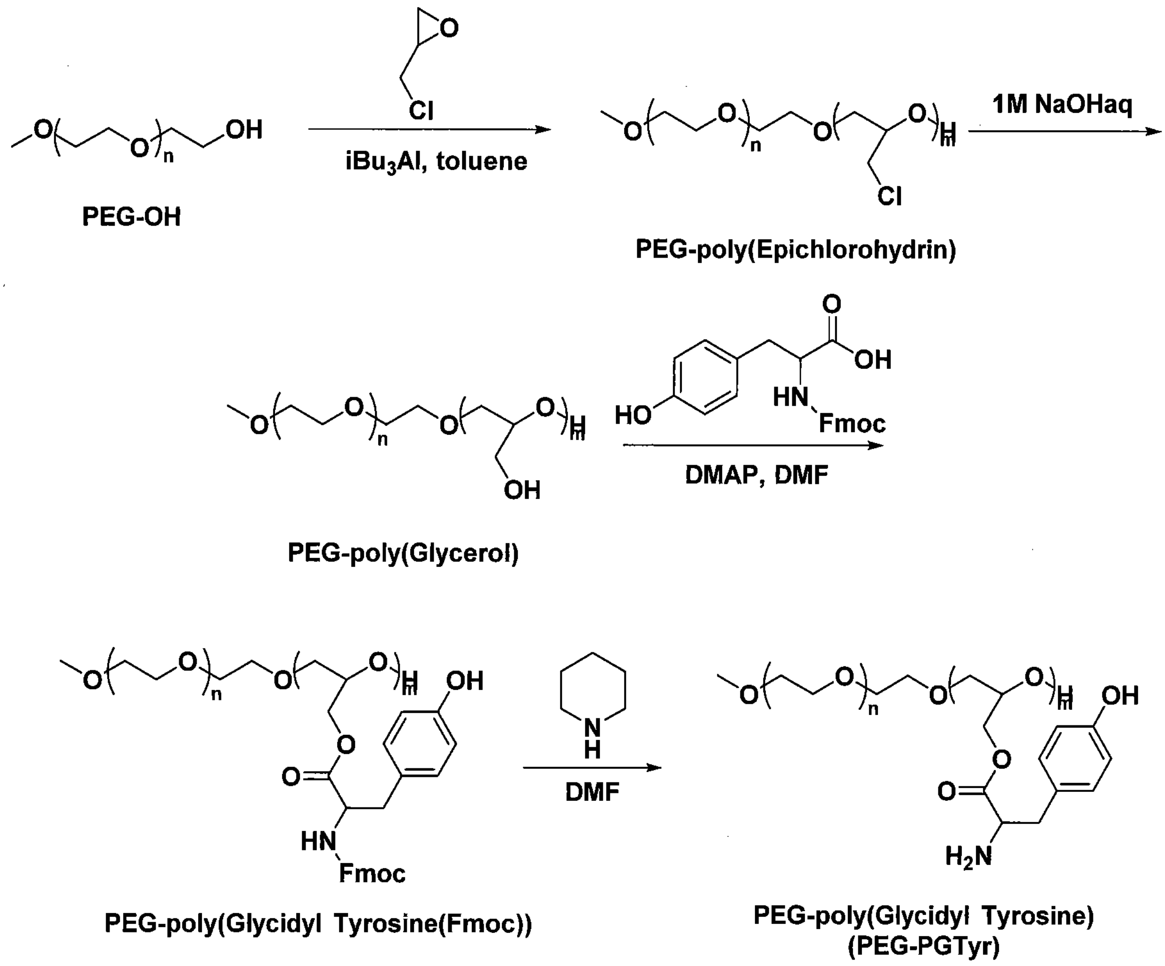


PEG-OH

PEG-poly(Epichlorohydrin)

PEG-poly(Glycerol)

PEG-poly(Glycidyl Tyrosine(Fmoc))

PEG-poly(Glycidyl Tyrosine) (PEG-PGTyr)

Polyethylene glycol having a hydroxyl group at the end was dissolved in toluene, and the resultant was heated

at 180°C for an hour to dehydrate the poly(ethylene glycol). To this, epichlorohydrin as a monomer and triisobutyl aluminum as a catalyst were added to allow polymerization at room temperature overnight. After the reaction, purification was conducted by reprecipitation with diethyl ether, which was followed by vacuum drying to give a powdery polymer. Next, an aqueous sodium hydroxide solution was added to allow reaction overnight, after which the resultant was purified by dialysis against water and then lyophilized to give a powdery polymer. The polymer was further dissolved in DMF, to which N-[(9H-fluorene-9-ylmethoxy)carbonyl]-L-tyrosine, dimethylaminopyridine and 1-(3-dimethylaminopropyl)-3-ethylcarbodiimide were added to allow reaction overnight. After the reaction, purification was conducted by reprecipitation with diethyl ether, which was followed by vacuum drying to give a powdery polymer. Next, the polymer was dissolved in DMF, to which piperidine was added to allow reaction overnight. After the reaction, the resultant was purified by dialysis against water and then lyophilized to give a powdery polymer. The resulting polymer (hereinafter, also referred to as PEG-PGTyr) was a polymer that could be represented by the following structural formula, where n = 272 and m = 81.

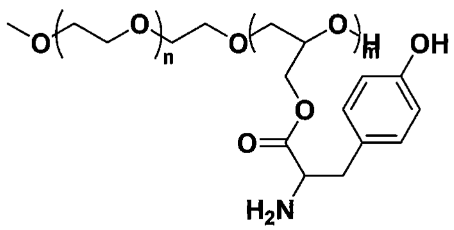

[Example 2]

<u>Tests for enzyme resistance in serum using PEG-PLL, PEG-PGBA, PEG-PGMA and PEG-PGLeu</u>

In this example, the stability of the polymeric micelles comprising PEG-PLL, PEG-PGBA, PEG-PGMA and PEG-PGLeu against enzymatic degradation was assessed using fetal bovine serum (FBS).

<Materials and method>

Micelles of PEG-PLL, PEG-PGBA, PEG-PGMA or PEG-PGLeu and luciferase mRNA were prepared by mixing PEG-PLL, PEG-PGBA, PEG-PGMA or PEG-PGLeu solution with mRNA solution at the N/P ratio of 3. Herein, the N/P ratio is defined by the following equation, and the same applies hereinafter unless otherwise specified.

$$\text{N/P ratio} = (\text{Total number of amino groups of polymer in solution})/(\text{Total number of phosphate groups of nucleic acids in solution})$$

FBS was diluted to be 50% with a 10 mM HEPES buffer (pH 7.3), to which the micelle solution was added and then left at 37°C for 15 minutes. Thereafter, RNeasy Mini Kit (QIAGEN) was used to extract mRNA from the solution. The extracted mRNA was reverse-transcribed to complementary DNA (cDNA), and the remaining cDNA was thereafter quantified by qRT-PCR. Relative values are shown, assuming that the amount of mRNA without leaving it at 37°C in the presence of FBS is 100%.

<Results>

As shown in Figure 1, the micelles comprising PEG-PGBA showed higher resistance against enzymatic degradation than the micelles comprising PEG-PLL. This implies that the enzyme resistance was enhanced by the flexible polyether segment of PEG-PGBA. Meanwhile, the enzyme resistance of the micelles comprising PEG-PGMA was comparable with that of the micelles comprising PEG-PGBA, which confirms that the number of the methylene groups in the side chain had no effect. Furthermore, the micelles comprising PEG-PGLeu showed higher enzyme resistance than the micelles comprising PEG-PGGly. This implies that the hydrophobic isobutyl group of PEG-PGLeu enhanced the enzyme resistance.

[Example 3]

Gene transfection into cultured cells using PEG-PLL. PEG-PGBA or PEG-PGMA

In this example, gene transfection capacity of the polymeric micelles comprising PEG-PLL, PEG-PGBA and PEG-PGMA were assessed with luciferase mRNA.

<Materials and method>

Micelles comprising PEG-PLL, PEG-PGBA or PEG-PGMA and luciferase mRNA were prepared by mixing PEG-PLL, PEG-PGBA or PEG-PGMA solution with a mRNA solution at the N/P ratio of 3.

Cells were prepared by seeding HuH-7 cells on a 96-well plate at 1.0 x $10^4$ cells/well, exchanging the medium with 10% FBS in DMEM (200 μL/well), and dropping each of the prepared micelle solutions into the medium to allow transfection. mRNA was administered in the amount of 200 ng/well. After 24 hours of incubation, luminescence intensity in the medium was determined.

<Results>

As shown in Figure 2, the micelles comprising PEG-PGBA showed higher gene expression level than the micelles comprising PEG-PLL. This implies that the flexible polyether segment of PEG-PGBA enhances the enzyme resistance to deliver mRNA to the cells. Meanwhile, the micelles comprising PEG-PGMA showed comparable gene expression efficiency with that of the micelles comprising PEG-PGBA, which confirms that the number of the methylene groups in the side chain had no effect.

[Example 4]

Tests for polyanion resistance in heparin using PEG-PLL, PEG-PGBA, PEG-PGMA, PEG-PGGly, PEG-PGLeu, PEG-PGTry and PEG-PGTyr

In this example, stabilities of the polymeric micelles comprising PEG-PLL, PEG-PGBA, PEG-PGMA, PEG-PGGly, PEG-PGLeu, PEG-PGTry and PEG-PGTyr against polyanion were assessed using heparin.

<Materials and method>

Micelles of PEG-PLL, PEG-PGBA, PEG-PGMA, PEG-PGGly, PEG-PGLeu, PEG-PGTry or PEG-PGTyr and fluorescently labeled luciferase mRNA were prepared by mixing PEG-PLL, PEG-PGBA, PEG-PGMA, PEG-PGGly, PEG-PGLeu, PEG-PGTry or PEG-PGTyr solution with mRNA solution at the N/P ratio of 3.

Heparin was diluted with 10 mM HEPES buffer (pH 7.3), and mixed with the micelle solutions at various S/P ratios and left at room temperature for 6 hours. Herein, the S/P ratio refers to the quantity defined by the following equation.

S/P ratio = (Total number of sulphate groups of heparin in solution)/(Total number of phosphate groups of nucleic acids in solution)

The micelle solution was diluted with 10 mM HEPES buffer (pH 7.3) to determine the diffusion coefficient of the fluorescence-labeled mRNA by fluorescence correlation spectroscopy.

<Results>

As shown in Figure 3, the micelles comprising PEG-PGBA showed higher polyanion resistance than the micelles comprising PEG-PLL. This implies that the polyanion resistance was enhanced by the flexible polyether segment of PEG-PGBA. Meanwhile, the diffusion coefficient of the micelles comprising PEG-PGMA was comparable with that of the micelle comprising PEG-PGBA, which confirms that the number of the methylene groups in the side chain had no effect on the polyanion resistance.

In addition, as shown in Figure 4, the micelle comprising PEG-PGLeu showed higher polyanion resistance than the micelles comprising PEG-PGGly. This implies that the polyanion resistance was enhanced by the hydrophobic isobutyl groups of PEG-PGLeu. In addition, the micelles comprising PEG-PGTry showed higher polyanion resistance

than that of the micelles comprising PEG-PGGly. This implies that polyanion resistance was enhanced by interaction of PEG-PGTry with the purine bases of the mRNA. Furthermore, the micelles comprising PEG-PGTyr showed higher polyanion resistance than that of the micelles comprising PEG-PGGly. This implies that polyanion resistance was enhanced by interaction of PEG-PGTry with the pyrimidine bases of the mRNA.

[Example 5]

Tests for *in vivo* stability using PEG-PLL, PEG-PGBA and PEG-PGMA

In this example, *in vivo* stabilities of the polymeric micelles comprising PEG-PLL, PEG-PGBA and PEG-PGMA were assessed by checking the blood circulation in mice.

<Materials and method>

Micelles of PEG-PLL, PEG-PGBA or PEG-PGMA and luciferase mRNA were prepared by mixing a PEG-PLL, PEG-PGBA or PEG-PGMA solution with a mRNA solution to a N/P ratio of 3.

200 μL of the micelle solution containing 40 μg of mRNA was administered *via* the tail vein of a Balb/C mouse. 2.5, 5 and 10 minutes later, 2 μL of blood was collected from the tail vein. mRNA was extracted from the collected blood by using RNeasy Mini Kit (QIAGEN). The extracted mRNA was reverse-transcribed to cDNA to quantify the cDNA by qRT-PCR.

<Results>

As shown in Figure 5, the micelles comprising PEG-PGBA showed better retention in blood than the micelles comprising PEG-PLL. This implies that the enzyme resistance and the polyanion resistance and thus the *in vivo* stability were enhanced by the flexible polyether segment of PEG-PGBA. Meanwhile, the micelles comprising PEG-PGMA showed similar retention in blood to that of the micelles comprising PEG-PGBA, which confirms that the number of the methylene groups in the side chain had no effect on the retention in blood.

[Example 6]

Hydrolysis tests using PEG-PLL and PEG-PGLeu

In this example, the kinetics of hydrolysis of PEG-PLL and PEG-PGLeu under physiological salt conditions were assessed by quantifying the primary amines in the polymers by fluorescamine assay.

<Materials and method>

PEG-PLL and PEG-PGLeu were each dissolved in PBS (pH 7.4) and left at 37°C for 1, 3, 6 and 24 hours. Thereafter, L-leucine released by hydrolysis was removed by ultrafiltration, and the resultant was subjected to lyophilization to give a powdery polymer.

The polymer was dissolved in PBS, to which fluorescamine dissolved in PBS was added to measure the fluorescence intensity. The fluorescence intensity was converted into the number of the primary amines based on N-butylamine to determine the number of the primary amines in the polymer.

<Results>

As shown in Figure 6, while no change was observed in the number of primary amines with PEG-PLL, the number of the primary amines decreased with PEG-PGLeu. This implies that PEG-PGLeu was hydrolyzed into safe poly(ethylene glycol)-block-polyglycerol (hereinafter, also referred to as PEG-PGlycerol) and L-leucine under physiological conditions by cleavage of the ester bond.

[Example 7]

Tests for the cytotoxicity against cultured cells using PEG-PLL and PEG-PGLeu

In this example, the cytotoxicity of PEG-PLL and PEG-PGLeu against cultured cells were assessed by the cell survival rates following addition of the polymer.

<Materials and method>

PEG-PLL and PEG-PGLeu were each dissolved in a 10 mM HEPES buffer (pH 7.3) to make serial dilutions of each polymer solution.

Cells were prepared by seeding HEK293 cells on a 96-well plate at 1.0 x $10^4$ cells/well, exchanging the medium with 10% FBS in DMEM (200 μL/well), and dropping each polymer solution into the medium. After 24 hours of incubation, the medium was exchanged with 10% FBS in DMEM to determine the cell survival number by the assay using Cell counting kit-8 (CCK-8). The cell survival rate was calculated, assuming that the cell survival rate where 10 mM HEPES buffer was added instead of the polymer solution was 100%.

<Results>

As shown in Figure 7, while the decrease in the cell survival rate was confirmed with PEG-PLL by addition of the polymer solution at a high concentration, no decrease in the cell survival rate was confirmed with PEG-PGLeu by addition of the polymer solution at a high concentration. This implies that PEG-PGLeu was hydrolyzed in the medium into safe PEG-PGlycerol and L-leucine, by which the positive charge density in the polymer and thus the cellular toxicity were reduced.

[Example 8]

Characterization of thermodynamic parameters of mRNA micelles using PEG-PGBA and PEG-PLL

In this example, thermodynamic parameters of the polymeric micelles comprising PEG-PGBA and PEG-PLL were determined by isothermal titration calorimetry (ITC).

Experimental method

PEG-PGBA or PEG-PLL solution (110 μM, 10 mM HEPES buffer (pH 7.3)) was dropped into mRNA solution (11 μM, 10 mM HEPES buffer (pH 7.3)) to observe the heat of reaction associated with PIC formation using iTC200 (GE Healthcare). In addition, the acquired measurement data was analyzed with ORIGIN7 software to calculate the binding ratio, the enthalpy, the entropy and the binding constant.

Results

As shown in Figure 8 and Table 1, the micelles comprising PEG-PGBA showed less change in the enthalpy and more change in the entropy than those of the micelles comprising PEG-PLL. This implies that the contact area with mRNA increased and the surface energy decreased due to the flexible PGBA chain, and that the formation of ion pairs with the mRNA and the release of free water were promoted. As a result, the binding constant between PEG-PGBA and mRNA was increased.

Table 1: Binding ratio and thermodynamic parameters of mRNA micelles

| Sample | Binding ratio | ΔH [kcal/mol] | ΔS [cal/(mol·K] | K [$M^{-1}$] |
|---|---|---|---|---|
| PEG-PLL/mRNA | 0.917 | 7.0 | 52.8 | $2.6\times10^6$ |
| PEG-PGBA/mRNA | 0.925 | 6.1 | 57.7 | $1.4\times10^8$ |

[Example 9]

Assessment of interaction with mRNA using PEG-PGTrp and PEG-PGTyr

In this example, interaction between the polymer and mRNA in each of the polymeric micelles comprising PEG-PGTrp and PEG-PGTyr were assessed by measuring the fluorescence intensity.

Experimental method

Micelles of PEG-PGTrp or PEG-PGTyr and luciferase mRNA were prepared by mixing PEG-PGTrp or PEG-PGTyr solution with mRNA solution at the N/P ratio of 3. In addition, micelles of PEG-PGTrp or PEG-PGTyr and Homo-poly(L-aspartate) (where the degrees of polymerization of Homo-PAsp was 80) were prepared by mixing PEG-PGTrp or PEG-PGTyr solution with Homo-PAsp solution at the N/P ratio of 3. Herein, similar to PEG-PLL, Homo-PAsp was synthesized by NCA polymerization using n-butylamine as an initiator.

The fluorescence intensities (Ex/Em = 280/350 nm) of the polymers and the prepared micelle solutions were determined with Nanodrop (Thermo Fisher Scientific, USA). The resulting fluorescence intensities were normalized assuming the fluorescence intensity of the polymer was 1.

Results

As shown in Figures 9 and 10, the fluorescence intensities decreased by addition of the mRNA to the polymers. This implies that tryptophan and tyrosine in the polymer undergo $\pi$-$\pi$ interaction with the base of the mRNA. Moreover, addition of base-free Homo-PAsp increased the fluorescence intensity. This implies that tryptophan and tyrosine in the polymer undergo $\pi$-$\pi$ interaction with a base of the mRNA.

[Example 10]

Tests for enzyme resistance in serum using PEG-PGLeu, PEG-PGTrp, PEG-PGTyr and PEG-PGGly

In this example, stabilities of the polymeric micelles comprising PEG-PGTrp and PEG-PGTyr against enzymatic attack were assessed using fetal bovine serum (FBS).

Experiment method

Micelles of PEG-PGLeu, PEG-PGTrp, PEG-PGTyr or PEG-PGGly and luciferase mRNA were prepared by mixing a PEG-PGLeu, PEG-PGTrp, PEG-PGTyr or PEG-PGGly solution with a mRNA solution to a N/P ratio of 3.

FBS was diluted to be 50% with 10 mM HEPES buffer (pH 7.3), to which the micelle solution was added and then left at 37°C for 15 minutes. Subsequently, RNeasy Mini Kit (QIAGEN) was used to extract mRNA from the solution. The extracted mRNA was reverse-transcribed to complementary DNA (cDNA), and the remaining cDNA was thereafter quantified by qRT-PCR. Relative values are shown, assuming that the amount of mRNA without leaving it at 37°C in the presence of FBS was 100%.

Results

As shown in Figure 11, the micelles comprising PEG-PGLeu showed better stability than the micelles comprising PEG-PGGly. This implies that enzyme resistance was enhanced by the hydrophobic isobutyl groups of PEG-PGLeu. In addition, the micelles comprising PEG-PGTrp and PEG-PGTyr showed better stability than the micelles comprising PEG-PGGly. This implies that enzyme resistance was enhanced by $\pi$-$\pi$ interaction of tryptophan or tyrosine with bases of mRNA.

[Example 11]

Gene transfection into cultured cells using PEG-PGLeu, PEG-PGTrp, PEG-PGTyr, PEG-PGGly

In this example, gene transfection capacity of the polymeric micelles comprising PEG-PGLeu, PEG-PGTrp, PEG-PGTyr and PEG-PGGly were assessed with luciferase mRNA.

Experimental method

Micelles of PEG-PGLeu, PEG-PGTrp, PEG-PGTyr or PEG-PGGly and luciferase mRNA were prepared by mixing a PEG-PGLeu, PEG-PGTrp, PEG-PGTyr or PEG-PGGly solution with a mRNA solution at the N/P ratio of 3.

Cells were prepared by seeding HuH-7 cells on a 96-well plate at 1.0 x $10^4$ cells/well, exchanging the medium with 10% FBS in DMEM (200 $\mu$L/well), and dropping each of the prepared micelle solutions into the medium to allow transfection. mRNA was administered in the amount of 200 ng/well. After 24 hours of incubation, luminescence intensity in the medium was determined.

Results

As shown in Figure 12, the micelles comprising PEG-PGLeu showed higher gene expression efficiency than

the micelles comprising PEG-PGGly. This implies that the enzyme resistance and the polyanion resistance were enhanced by the hydrophobic isobutyl groups of PEG-PGLeu, and by which the stability under the culture conditions was enhanced. Meanwhile, the micelles comprising PEG-PGTrp and PEG-PGTyr showed better stability than the micelles comprising PEG-PGGly. This implies that the enzyme resistance and the polyanion resistance were enhanced by π-π interaction of tryptophan or tyrosine with bases of the mRNA, enhancing stability under the cell culture conditions.

[Example 12]

Tests for *in vivo* stability using PEG-PGLeu, PEG-PGTrp, PEG-PGTyr and PEG-PGGly

In this example, *in vivo* stabilities of the polymeric micelles comprising PEG-PGLeu, PEG-PGTrp, PEG-PGTyr and PEG-PGGly were assessed by observing the blood circulation in mice.

Experimental method

Micelles of PEG-PGLeu, PEG-PGTrp, PEG-PGTyr or PEG-PGGly and luciferase mRNA were prepared by mixing a PEG-PGLeu, PEG-PGTrp, PEG-PGTyr of PEG-PGGly solution with mRNA solution at the N/P ratio of 3.

200 μL of the micelle solution containing 40 μg of mRNA was administered *via* the tail vein of a Balb/C mouse. 2.5, 5 and 10 minutes later, 2 μL of blood was collected from the tail vein. mRNA was extracted from the collected blood by using RNeasy Mini Kit (QIAGEN). The extracted mRNA was reverse-transcribed to cDNA to quantify the cDNA by qRT-PCR.

Results

As shown in Figure 13, the micelles comprising PEG-PGLeu showed better retention in blood than the micelles comprising PEG-PGGly. This implies that the enzyme resistance and the polyanion resistance and thus the *in vivo* stability were enhanced by the hydrophobic isobutyl groups of PEG-PGLeu. Meanwhile, the micelles comprising PEG-PGTrp and PEG-PGTyr showed better retention in blood than the micelles comprising PEG-PGGly. This implies that the enzyme resistance and the polyanion resistance and thus the *in vivo* stability were enhanced by π-π interaction of tryptophan or tyrosine with bases of the mRNA.

[Example 13]

*In vivo* gene transfection using PEG-PGBA and PEG-PLL

In this example, efficiencies of *in vivo* gene transfection of the polymeric micelles comprising PEG-PGBA and PEG-PLL were assessed by the luciferase expression efficiency in mouse lungs.

Experimental method

Micelles of PEG-PGBA or PEG-PLL and luciferase mRNA were prepared by mixing PEG-PGBA or PEG-PLL solution with mRNA solution at the N/P ratio of 3.

200 μL of the micelle solution containing 67 μg of mRNA was intratracheally administered to a Balb/C mouse. 24 hours later, the lung was removed. The removed lung was added to cell lysis buffer (1x passive lysis buffer, Promega, USA) to homogenize using Multi-beads shocker (Yasui Kikai Corporation) to determine the luminescence intensity in the solution.

Results

As shown in Figure 14, the micelles comprising PEG-PGBA and PEG-PLL showed better gene expression efficiencies than the group administered with mRNA alone. This implies that encapsulation of the mRNA in the polymeric micelles enhanced the enzyme resistance and the polyanion resistance and thus the efficiency of *in vivo* gene transfection. Furthermore, the micelles comprising PEG-PGBA showed better gene expression efficiency than the micelles comprising PEG-PLL. This implies that the enzyme resistance and the polyanion resistance and thus the efficiency of *in vivo* gene transfection were enhanced by the polyether backbone of PEG-PGBA.

[Example 14]

*In vivo* gene transfection using PEG-PGTrp and PEG-PGGly

In this example, efficiency of *in vivo* gene transfection of the polymeric micelles comprising PEG-PGTrp and PEG-PGGly were assessed by efficiency of luciferase expression in mouse lungs.

Experimental method

Micelles of PEG-PGTrp or PEG-PGGly and luciferase mRNA were prepared by mixing PEG-PGTrp or PEG-PGGly solution with mRNA solution at the N/P ratio of 3.

200 μL of the micelle solution containing 67 μg of mRNA was intratracheally administered to a Balb/C mouse. 24 hours later, the lung was removed. The removed lung was added to cell lysis buffer (lx passive lysis buffer, Promega, USA) to homogenize using Multi-beads shocker (Yasui Kikai Corporation) to determine the luminescence intensity in the solution.

Results

As shown in Figure 15, the micelles comprising PEG-PGTrp showed better gene expression efficiency than the micelles comprising PEG-PGGly. This implies that π-π interaction between tryptophan of PEG-PGTrp and bases of the mRNA enhanced the enzyme resistance and the polyanion resistance and thus the efficiency of *in vivo* gene transfection.

[Example 15]

Hydrolysis tests using PEG-PGLeu, PEG-PGTrp, PEG-PGTyr and PEG-PGGly

In this example, kinetics of hydrolysis of PEG-PGLeu, PEG-PGTrp, PEG-PGTyr and PEG-PGGly under physiological salt conditions were assessed by quantifying the primary amines in the polymers by fluorescamine assay.

Experiment method

PEG-PGLeu, PEG-PGTrp, PEG-PGTyr and PEG-PGGly were each dissolved in PBS (pH 7.4) and left at 37°C for 1, 3, 6 and 24 hours. Thereafter, L-leucine released by hydrolysis was removed by ultrafiltration, and the resultant was subjected to lyophilization to give a powdery polymer.

The polymer was dissolved in PBS, to which fluorescamine in PBS was added to determine the fluorescence intensity. The fluorescence intensity was converted into the number of the primary amines based on N-butylamine to determine the number of the primary amines in the polymer.

Results

As shown in Figure 16, while no change was found in the number of primary amines with PEG-PLL, the number of the primary amines decreased with PEG-PGLeu, PEG-PGTrp, PEG-PGTyr and PEG-PGGly. This implies that these polymers were hydrolyzed into safe poly(ethylene glycol)-block-polyglycerol and the amino acid under physiological conditions by cleavage of the ester bond.

[Example 16]

Tests for the cytotoxicity against cultured cells using PEG-PGLeu, PEG-PGTrp, PEG-PGTyr and PEG-PGGly

In this example, the cytotoxicity of PEG-PGLeu, PEG-PGTrp, PEG-PGTyr and PEG-PGGly against cultured cells were assessed by the cell survival rates following addition of the polymer.

Experimental method

PEG-PGLeu, PEG-PGTrp, PEG-PGTyr and PEG-PGGly were dissolved in 10 mM HEPES buffer (pH 7.3) to make serial dilutions of each polymer solution.

Cells were prepared by seeding HEK293 cells on a 96-well plate at 1.0 x $10^4$ cells/well, exchanging the medium

with 10% FBS in DMEM (200 µL/well), and dropping each polymer solution into the medium. After 24 hours of incubation, the medium was exchanged with 10% FBS in DMEM to determine the cell survival number by an assay using Cell counting kit-8 (CCK-8). The cell survival rate was calculated, assuming that the cell survival rate where 10 mM HEPES buffer was added instead of the polymer solution was 100%.

Results

As shown in Figure 17, while the decrease in the cell survival rate was confirmed with PEG-PLL by addition of the polymer solution at a high concentration, no decrease in the cell survival rates was confirmed with PEG-PGLeu, PEG-PGTrp, PEG-PGTyr and PEG-PGGly by addition of the polymer solutions even at a high concentration. This implies that PEG-PGLeu, PEG-PGTrp, PEG-PGTyr and PEG-PGGly were hydrolyzed in the medium into safe PEG-PGlycerol and the amino acid, by which the positive charge density in the polymer and thus the cellular toxicity were reduced.

## Claims

1. A polymer compound selected from a homopolymer of (a) and a block copolymer of (b) below:

   (a) a homopolymer comprising a polyether segment having a side chain containing a primary amine;
   (b) a block copolymer comprising a polyether segment having a side chain containing a primary amine and a poly(ethylene glycol) chain.

2. The polymer compound according to Claim 1, wherein the homopolymer is represented by Formula I below:

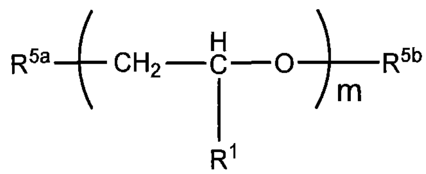

(I)

(wherein, $R^1$ represents a primary amine linked *via* a methylene group or an ester bond, $R^{5a}$ and $R^{5b}$ each independently represent a hydrogen atom, a halogen atom or a group represented by $-OCOCH(NH_2)R^3$ (wherein, $R^3$ represents a hydrogen atom or a group represented by:

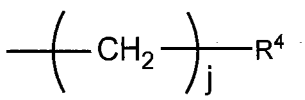

(wherein, $R^4$ represents an optionally substituted hydrocarbon group having a carbon number of 1-20 or an optionally substituted heterocyclic functional group, and j represents an integer of 1-5),
and m represents an integer of 2-5,000).

3. The polymer compound according to Claim 2, wherein the primary amine linked *via* a methylene group or an ester bond is represented by the following formula:

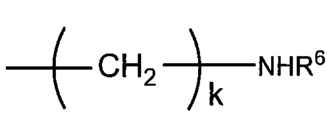

or

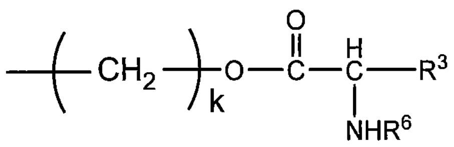

(wherein, $R^3$ represents a hydrogen atom or a group represented by:

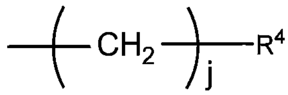

(wherein, $R^4$ represents an optionally substituted hydrocarbon group having a carbon number of 1-20 or an optionally substituted heterocyclic functional group, and j represents an integer of 1-5), or $R^3$ and $R^4$ each independently represent a side chain structure of an arbitrary amino acid of a protein, $R^6$ represents a hydrogen atom or one or more arbitrary amino acids, and k represents an integer of 1-5).

4. The polymer compound according to Claim 1, wherein the block copolymer is represented by Formula II below:

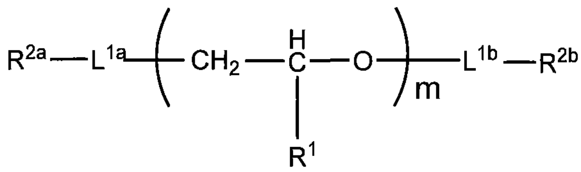

(II)

[wherein, $R^1$ represents a primary amine linked *via* a methylene group or an ester bond, $R^{2a}$ and $R^{2b}$ each independently represent a hydrogen atom or a group represented by:

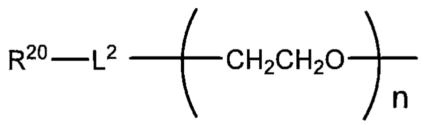

(wherein, $R^{20}$ represents a group represented by $-O-CH_3$ or a ligand molecule, $L^2$ represents a linking group, and n represents an integer of 5-20,000), $L^{1a}$ and $L^{1b}$ each independently represent a linking group, and m represents an integer of 2-5,000].

5. The polymer compound according to Claim 4, wherein the primary amine linked *via* a methylene group or an ester bond is represented by the following formula:

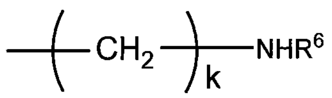

or

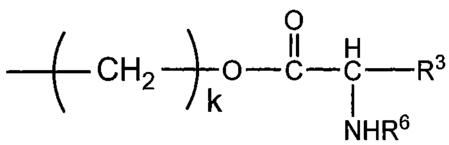

(wherein, $R^3$ represents a hydrogen atom or a group represented by:

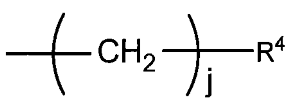

(wherein, $R^4$ represents an optionally substituted hydrocarbon group having a carbon number of 1-20 or an optionally substituted heterocyclic functional group, and j represents an integer of 1-5), or $R^3$ and $R^4$ each independently represent a side chain structure of an arbitrary amino acid of a protein, $R^6$ represent a hydrogen atom or one or more arbitrary amino acids, and k represents an integer of 1-5).

6. The compound according to any one of Claims 2-5, wherein $R^1$ is represented by $-CH_2NH_2$ or $-CH_2CH_2CH_2CH_2NH_2$.

7. The compound according to any one of Claims 2-5, wherein $R^1$ is represented by:

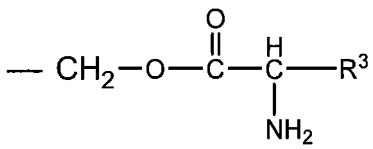

(wherein, $R^3$ represents a hydrogen atom or a group represented by:

$-CH_2-R^4$

(wherein, $R^4$ represents a propyl group, an optionally substituted indolyl group or an optionally substituted phenyl group).

**8.** The compound according to any one of Claims 4-7, wherein $L^{1a}$ and/or $L^{1b}$ is represented by $-CH_2CH_2O-$.

**9.** The compound according to any one of Claims 4-8, wherein $R^2$ is represented by:

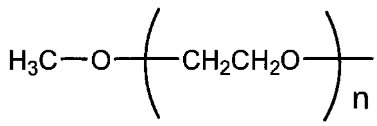

(wherein, n represents an integer of 5-20,000).

**10.** A polyion complex comprising the polymer compound according to any one of Claims 1-7 and nucleic acids.

**11.** The polyion complex according to Claim 10, wherein the nucleic acids are RNA or DNA.

**12.** The polyion complex according to Claim 11, wherein RNA is siRNA, mRNA, antisense RNA, RNA aptamer, self-replicating RNA, miRNA (micro RNA) or lncRNA (long non-coding RNA).

**13.** The polyion complex according to Claim 11, wherein DNA is antisense DNA, DNA aptamer, pDNA (plasmid DNA) or MCDNA (minicircle DNA).

**14.** A kit for preparing a device to deliver nucleic acids to targeted cells or tissues, the kit comprising the polymer compound according to any one of Claims 1-9.

**15.** A device for delivering nucleic acids to target cells or tissues, the device comprising the polyion complex according to any one of Claims 10-13.

**16.** A method for producing a block copolymer comprising a polyether segment having a side chain containing a primary amine and a poly(ethylene glycol) chain, the method comprising: obtaining a block copolymer comprising poly(ethylene glycol) and a polyether segment having a side chain by dehydrating poly(ethylene glycol) and then carrying out epoxy ring-opening polymerization; and introducing a primary amine into the side chain of the polyether segment.

**17.** The method according to Claim 16, wherein dehydration is performed using toluene.

**18.** The method according to either one of Claims 16 and 17, wherein epoxy ring-opening polymerization is carried out using 1,2-epoxy-5-hexene or epichlorohydrin.

Fig. 1

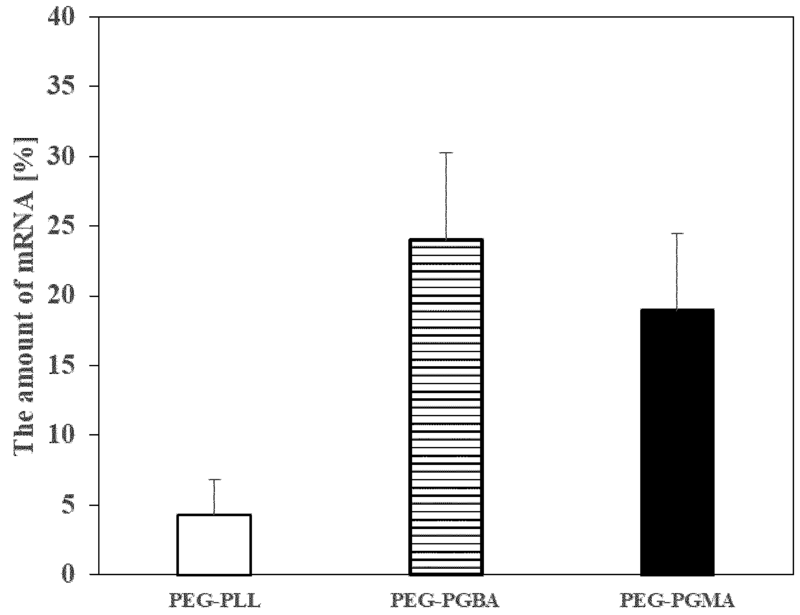
The amount of mRNA [%]
40
35
30
25
20
15
10
5
0
PEG-PLL
PEG-PGBA
PEG-PGMA

Fig. 2

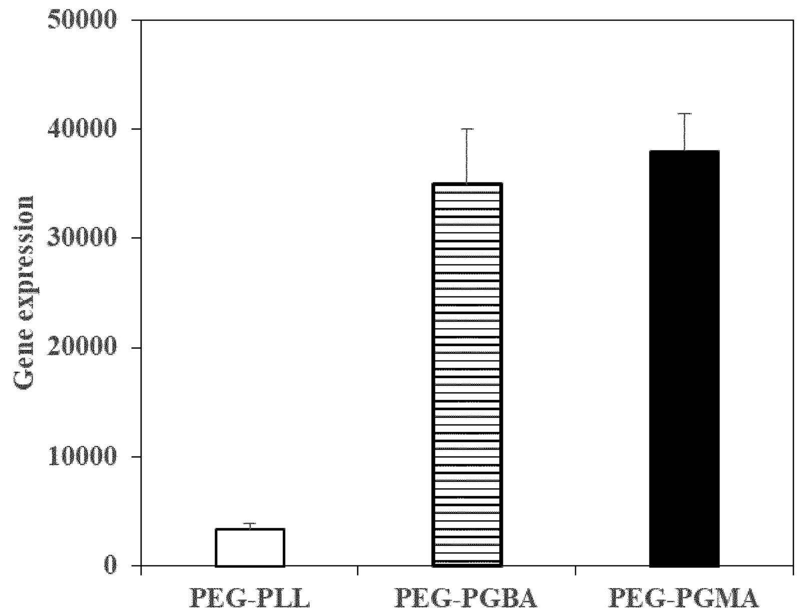
Gene expression
50000
40000
30000
20000
10000
0
PEG-PLL
PEG-PGBA
PEG-PGMA

Fig. 3

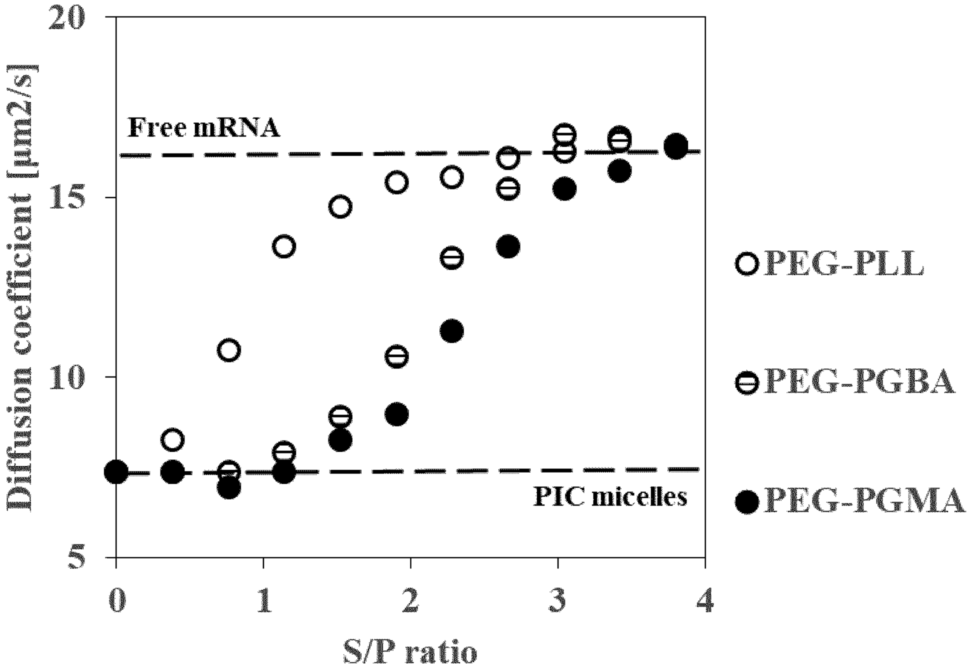
Diffusion coefficient [μm2/s]
20
15
10
5
0
1
2
3
4
S/P ratio
Free mRNA
PIC micelles
PEG-PLL
PEG-PGBA
PEG-PGMA

Fig. 4

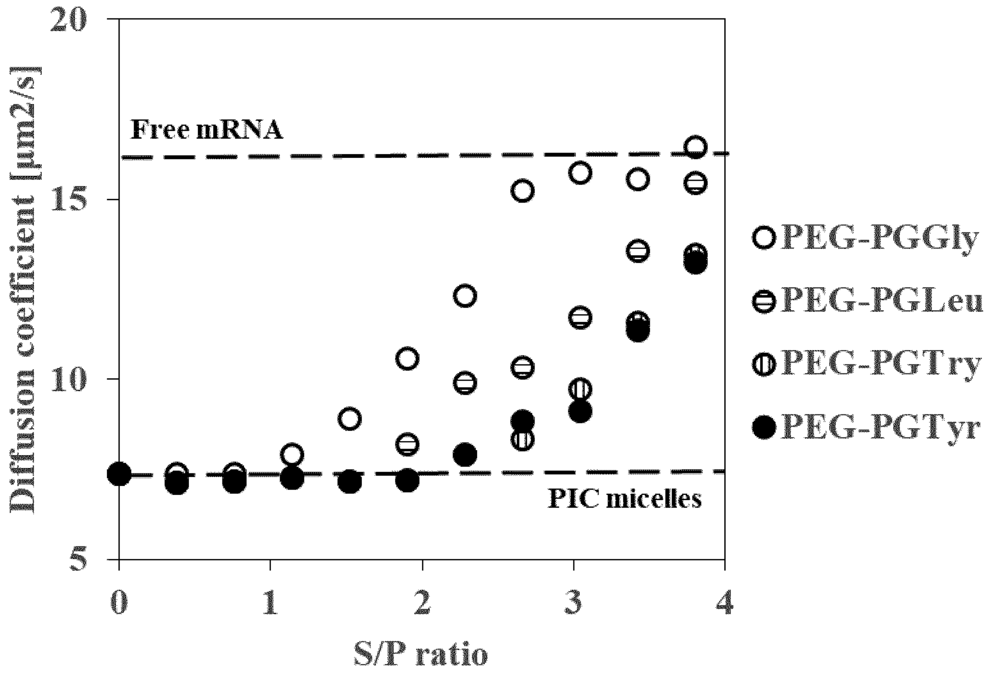
20
15
10
5
0
1
2
3
4
Diffusion coefficient [µm2/s]
S/P ratio
Free mRNA
PIC micelles
PEG-PGGly
PEG-PGLeu
PEG-PGTry
PEG-PGTyr

Fig. 5

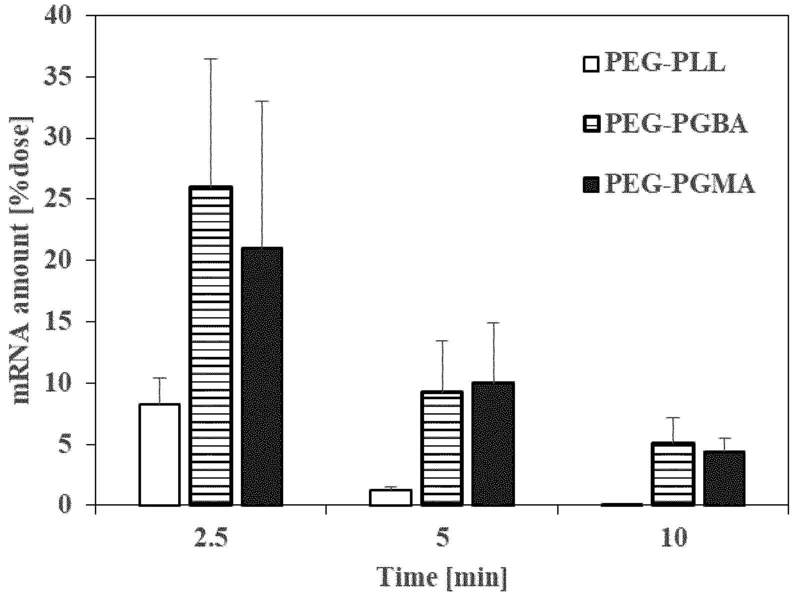
40
35
30
25
20
15
10
5
0
mRNA amount [%dose]
PEG-PLL
PEG-PGBA
PEG-PGMA
2.5
5
10
Time [min]

Fig. 6

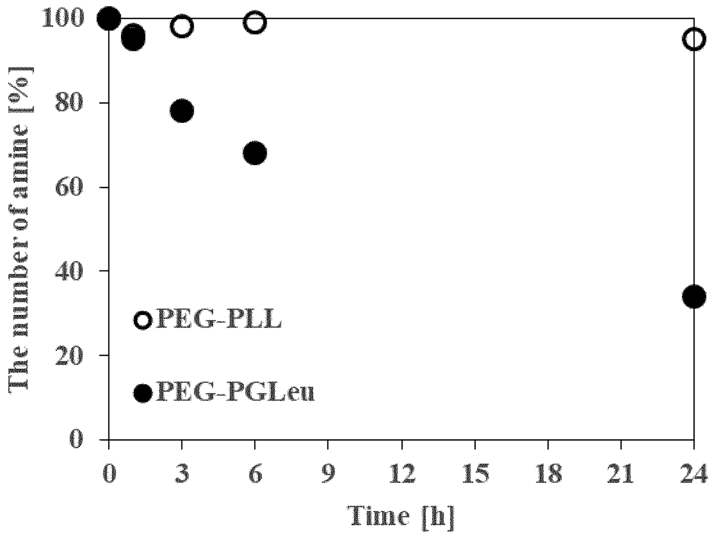
100
80
60
40
20
0
The number of amine [%]
PEG-PLL
PEG-PGLeu
0
3
6
9
12
15
18
21
24
Time [h]

Fig. 7

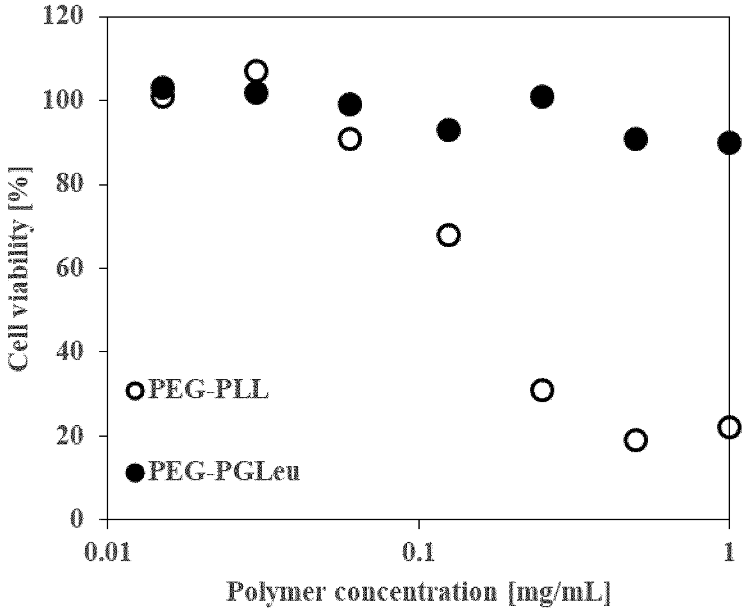
120
100
80
60
40
20
0
Cell viability [%]
PEG-PLL
PEG-PGLeu
0.01
0.1
1
Polymer concentration [mg/mL]

Fig. 8

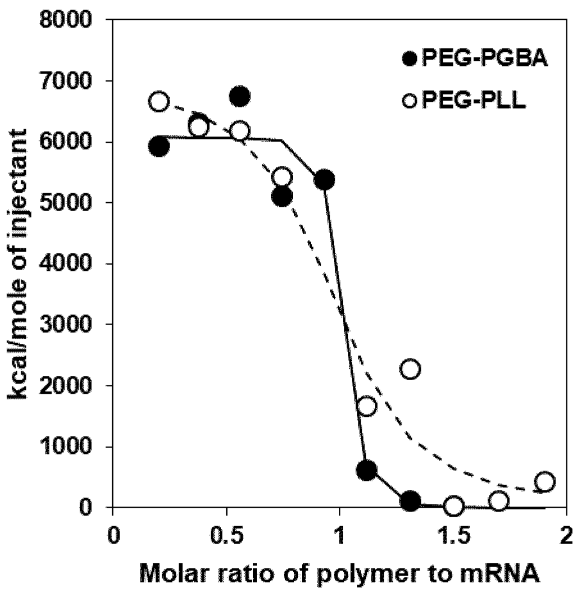
8000
7000
6000
5000
4000
3000
2000
1000
0
kcal/mole of injectant
PEG-PGBA
PEG-PLL
0
0.5
1
1.5
2
Molar ratio of polymer to mRNA

Fig. 9

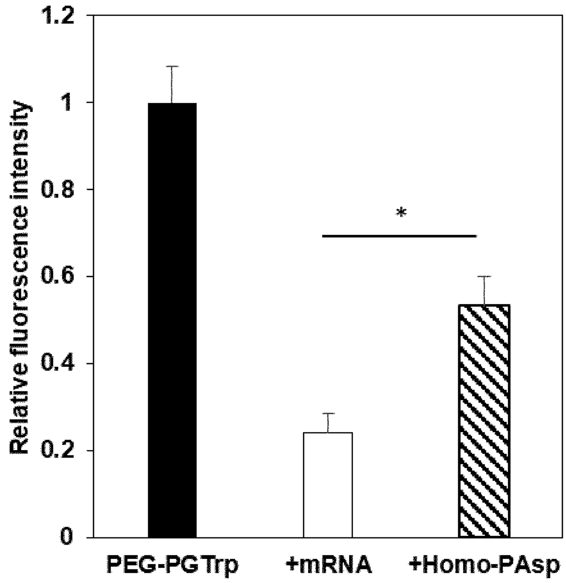
1.2
1
0.8
0.6
0.4
0.2
0
Relative fluorescence intensity
*
PEG-PGTrp
+mRNA
+Homo-PAsp

Fig. 10

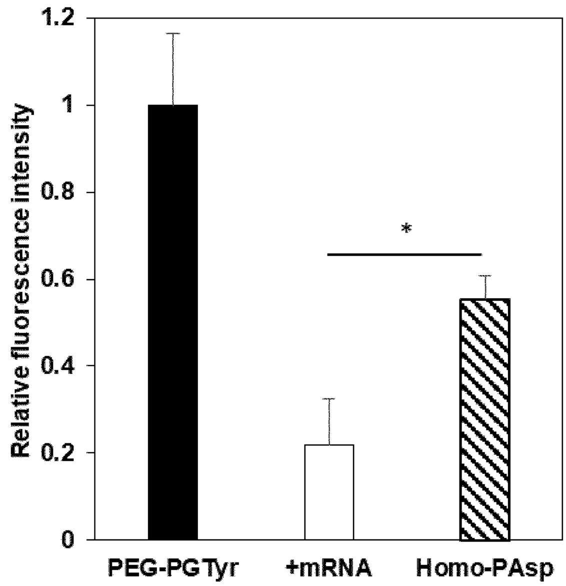
1.2
1
0.8
0.6
0.4
0.2
0
Relative fluorescence intensity
*
PEG-PGTyr
+mRNA
Homo-PAsp

Fig. 11

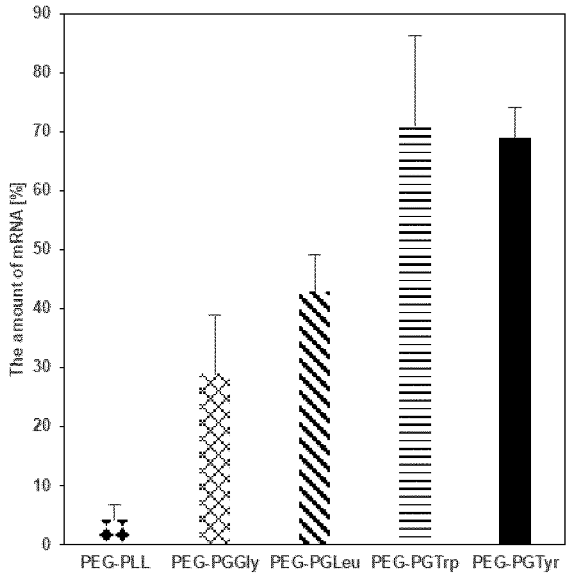
90
80
70
60
50
40
30
20
10
0
The amount of mRNA [%]
PEG-PLL
PEG-PGGly
PEG-PGLeu
PEG-PGTrp
PEG-PGTyr

Fig. 12

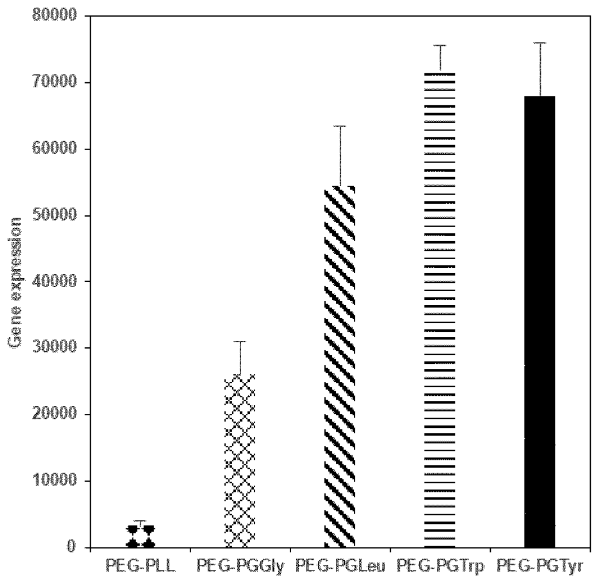
80000
70000
60000
50000
40000
30000
20000
10000
0
Gene expression
PEG-PLL
PEG-PGGly
PEG-PGLeu
PEG-PGTrp
PEG-PGTyr

Fig. 13

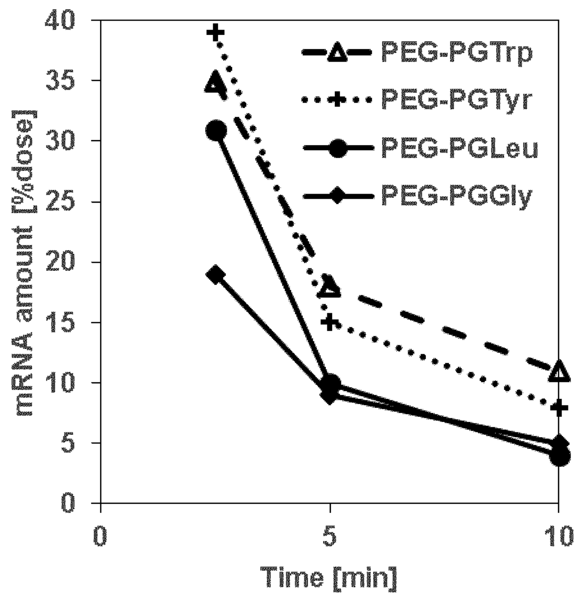
40
35
30
25
20
15
10
5
0
mRNA amount [%dose]
0
5
10
Time [min]
PEG-PGTrp
PEG-PGTyr
PEG-PGLeu
PEG-PGGly

Fig. 14

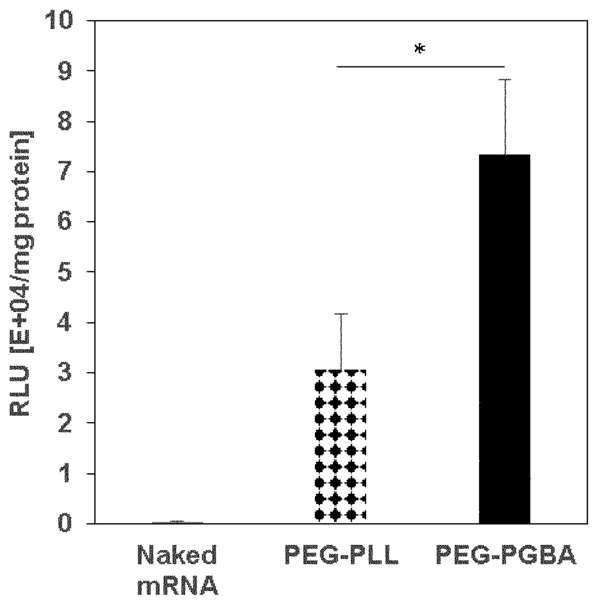
10
9
8
7
6
5
4
3
2
1
0
RLU [E+04/mg protein]
*
Naked mRNA
PEG-PLL
PEG-PGBA

Fig. 15

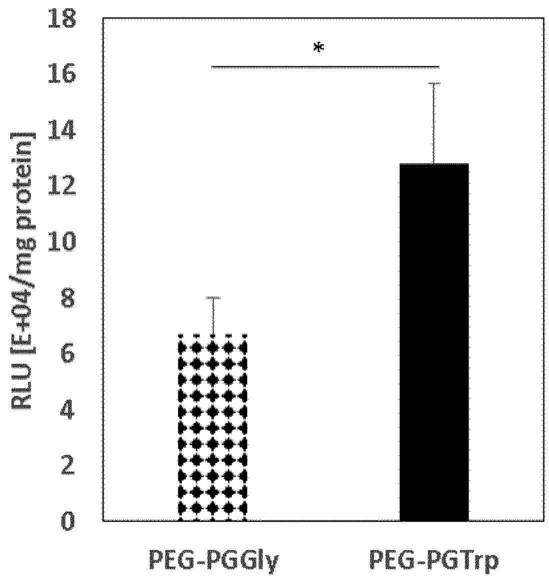
18
16
14
12
10
8
6
4
2
0
RLU [E+04/mg protein]
*
PEG-PGGly
PEG-PGTrp

Fig. 16

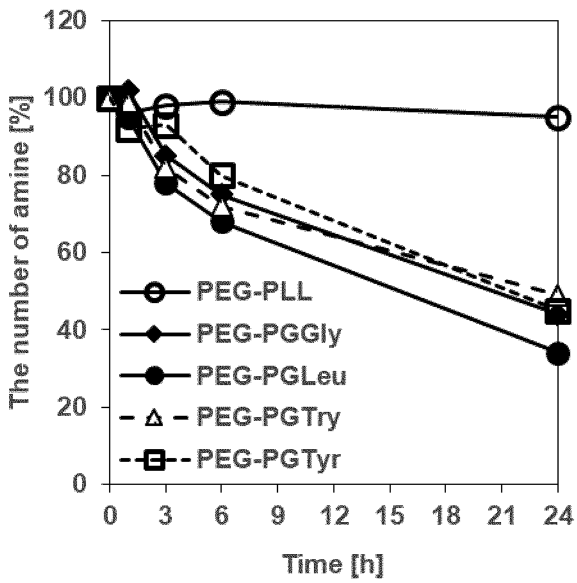
120
100
80
60
40
20
0
The number of amine [%]
PEG-PLL
PEG-PGGly
PEG-PGLeu
PEG-PGTry
PEG-PGTyr
0
3
6
9
12
15
18
21
24
Time [h]

Fig. 17

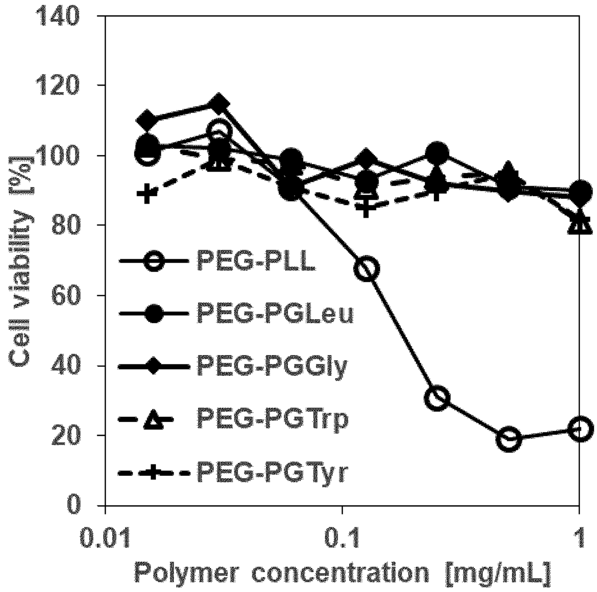
140
120
100
80
60
40
20
0
Cell viability [%]
PEG-PLL
PEG-PGLeu
PEG-PGGly
PEG-PGTrp
PEG-PGTyr
0.01
0.1
1
Polymer concentration [mg/mL]

**INTERNATIONAL SEARCH REPORT**

International application No. PCT/JP2019/003617

A. CLASSIFICATION OF SUBJECT MATTER
Int.Cl. C08G65/28(2006.01)i, A61K31/7088(2006.01)i, A61K47/34(2017.01)i, C08G65/328(2006.01)i

According to International Patent Classification (IPC) or to both national classification and IPC

B. FIELDS SEARCHED

Minimum documentation searched (classification system followed by classification symbols)
Int.Cl. C08G65/28, A61K31/7088, A61K47/34, C08G65/328

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | US 2017/0218219 A1 (ENNIS PAINT, INC.) 03 August 2017, claims & US 2018/0163077 A1 | 1<br>2-18 |
| X<br>A | O'CONNOR, Aoife et al., "Poly(Ethylene Glycol)-Based Backbones with High Peptide Loading Capacities", molecules, 30 October 2014, 19, 17559-17577 | 1<br>2-18 |
| A | CN 103936973 A (CHINESE ACAD INST CHEMISTRY) 23 July 2014 (Family: none) | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 April 2019 (15.04.2019) | 23 April 2019 (03.04.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/003617

C (Continuation). DOCUMENTS CONSIDERED TO BE RELEVANT

| Category* | Citation of document, with indication, where appropriate, of the relevant passages | Relevant to claim No. |
|---|---|---|
| A | MEYER, Joerg et al., "Poly(glycidyl amine) and Copolymers with Glycidol and Glycidyl Amine Repeating Units: Synthesis and Characterization", Macromolecules, 44, 06 May 2011, 4082-4091 | 1-18 |
| A | WO 2012/005376 A1 (THE UNIVERSITY OF TOKYO) 12 January 2012 & US 2013/0109743 A1 & EP 2591792 A1 & CN 102971002 A | 1-18 |
| A | JP 2009-149728 A (THE NOGUCHI INSTITUTE) 09 July 2009 (Family: none) | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **U. SAHIN.** *Nat. Rev. Drug Discovery,* 2014, vol. 13, 759-780
- **N. B. TSUI.** *Clin. Chem.,* 2002, vol. 48, 1647-1653
- **S. UCHIDA.** *Biomaterials,* 2016, vol. 82, 221-228
- **K. HAYASHI.** *Macromol. Rapid Commun.,* 2016, vol. 37, 486-493